(19) Europäisches Patentamt
European Patent Office
Office européen des brevets

(11) Publication number: 0 601 459 A2

# (12) EUROPEAN PATENT APPLICATION

(21) Application number: **93119356.9**

(22) Date of filing: **01.12.93**

(51) Int. Cl.5: **C07K 5/06**, A61K 37/64

(30) Priority: **02.12.92 US 984640**
**26.08.93 US 112153**

(43) Date of publication of application:
**15.06.94 Bulletin 94/24**

(84) Designated Contracting States:
**AT BE CH DE DK ES FR GB GR IE IT LI LU MC NL PT SE**

(71) Applicant: **BRISTOL-MYERS SOUIBB COMPANY**
**P.O. Box 4000**
**Princeton, NJ 08543-4000(US)**

(72) Inventor: **Kimball, Spencer D.**
**13 Charred Oak Lane**
**E. Windsor, NJ 08520(US)**
Inventor: **Das, Jagabandhu**
**38 Martin Lane**
**Mercerville, NJ 08619(US)**
Inventor: **Lau, Wan Fang**
**104 Ouince Court**
**Lawrenceville, NJ 08648(US)**

(74) Representative: **Josif, Albert, Dr.-Ing. et al**
**Baaderstrasse 3**
**D-80469 München (DE)**

(54) **Sulfonamido heterocyclic thrombin inhibitors.**

(57) Sulfonamido heterocyclic thrombin inhibitors are provided which have the structure

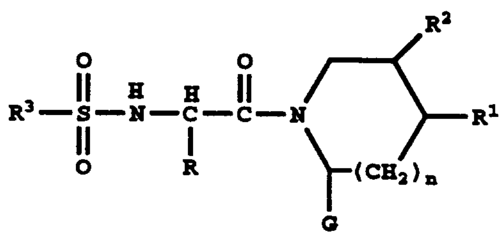

wherein G is

EP 0 601 459 A2

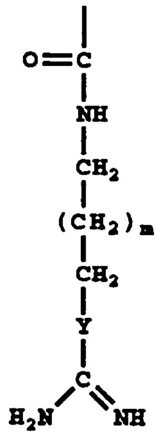

or

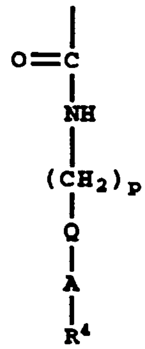

including all stereoisomers thereof and salts thereof, wherein n is 0, l or 2 or 3; m is 0, l, 2 or 3; Y is NH or S; p is 0, l or 2, Q is a single bond or

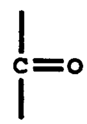

A is aryl, cycloalkyl, azacycloalkyl or azaheteroalkyl;

R is hydrogen, hydroxyalkyl, aminoalkyl, alkyl, cycloalkyl, amidoalkyl, arylalkyl, alkenyl, aryl, alkynyl, arylalkoxyalkyl, or an amino acid side chain;

$R^1$ and $R^2$ are independently hydrogen, lower alkyl, cycloalkyl, aryl, hydroxy, alkoxy, keto, thioketal, thioalkyl, thioaryl, amino or alkylamino; or $R^1$ and $R^2$ can be taken together with the carbons to which they are attached to form a cycloalkyl, aryl or heteroaryl ring;

$R^3$ is lower alkyl, aryl, arylalkyl, heteroaryl, quinolinyl or tetrahydroquinolinyl; and

$R^4$ can be guanidine, amidine or aminomethyl; where A includes a hetero atom, $R^4$ is amidino; otherwise it may be any of the $R^4$ moieties set out above.

This is a continuation-in-part of application Serial No. 112,153 filed August 26, 1993, which is a continuation-in-part of application Serial No. 984,640 filed December 2, 1992.

The present invention relates to sulfonamido heterocyclic compounds which are thrombin inhibitors and thus useful in inhibiting formation of thrombi.

The sulfonamido heterocyclic thrombin inhibitors of the invention have the structure I

I 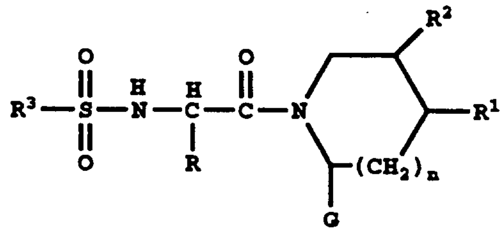

wherein G is an amido moiety which is

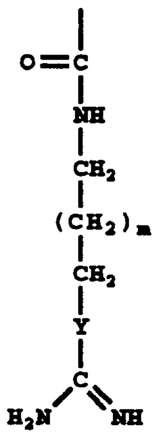

(G1)

or

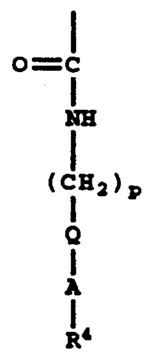

(G2)

including all stereoisomers thereof; and including all pharmaceutically acceptable salts thereof; wherein

R is hydrogen, hydroxyalkyl, aminoalkyl, amidoalkyl, alkyl, cycloalkyl, aryl, arylalkyl, alkenyl, alkynyl, arylalkoxyalkyl, or an amino acid side chain, either protected or unprotected;

$R^1$ and $R^2$ are independently hydrogen, lower alkyl, cycloalkyl, aryl, hydroxy, alkoxy, keto, thioketal, thioalkyl, thioaryl, amino or alkylamino; or $R^1$ and $R^2$ together with the carbons to which they are attached form a cycloalkyl, aryl, or heteroaryl ring; and

$R^3$ is lower alkyl, aryl, arylalkyl, heteroaryl, quinolinyl or tetrahydroquinolinyl;

n is 0, 1 or 2;

m is 0, 1, 2 or 3;

Y is NH or S;

p is 0, 1 or 2;

Q is a single bond or

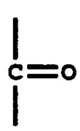

;

A is aryl or cycloalkyl, or an azacycloalkyl ring

A of 4 to 8 carbons in the ring or an azaheteroalkyl ring A of 4 to 8 carbons in the ring,

A

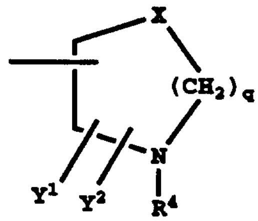

where X is $CH_2$, O, S or NH;

q is 0, l, 2, 3 or 4 if X is $CH_2$;

q is 2, 3 or 4 if x is O, S or NH;

$Y^1$ and $Y^2$ are independently H, lower alkyl, halo or keto; and

$R^4$ is guanidine, amidine or aminomethyl;

where A is aryl or cycloalkyl, $R^4$ is guanidine, amidine or aminomethyl;

where A is azacycloalkyl or azaheteroalkyl, $R^4$ is amidine;

provided that where X is a hetero atom (that is, A is azaheteroalkyl), then there must be at least a 2-carbon chain between X and any N atom in the ring A or outside ring A;

and provided that where G is GI, then if $R^3$ is alkyl, the alkyl must contain at least 3 carbons.

Examples of the A ring (azacycloalkyl, or azaheteroalkyl) which may be employed herein include

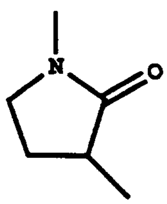 , 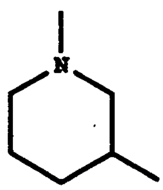 , 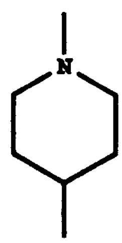 , 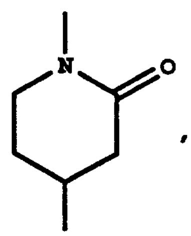 ,

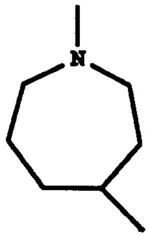 , 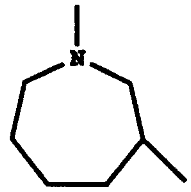 , 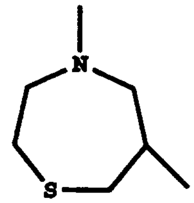 ,

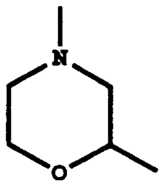 , 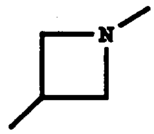 , 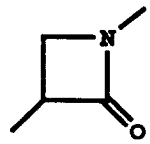 ,

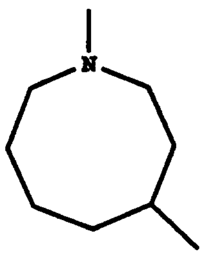 , 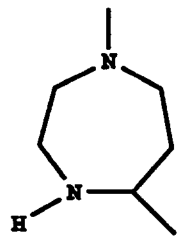 , 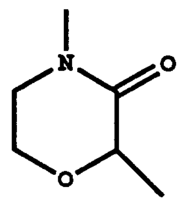

and the like.

The term "lower alkyl" or "alkyl" as employed herein by itself or as part of another group includes both straight and branched chain radicals of up to l8 carbons, preferably l to 8 carbons, such as methyl, ethyl, propyl, isopropyl, butyl, t-butyl, isobutyl, pentyl, hexyl, isohexyl, heptyl, 4,4-dimethylpentyl, octyl, 2,2,4-trimethylpentyl, nonyl, decyl, undecyl, dodecyl, the various branched chain isomers thereof, and the like as well as such groups including l, 2 or 3 halo substituents (for example, to form $CF_3$ or $CF_3CH_2$) and/or l or 2 of the following substituents: an aryl substituent (for example, to form benzyl or phenethyl), an alkyl-aryl substituent, a haloaryl substituent, a cyclo-alkyl substituent, an alkylcycloalkyl substituent, an alkenyl substituent, an alkynyl substituent, hydroxy or a carboxy substituent. It will be appreciated that the same "alkyl" group may be substituted with one or more of any of the above substituents.

The term "cycloalkyl" by itself or as part of another group includes saturated cyclic hydrocarbon groups containing 3 to l2 carbons, preferably 3 to 8 carbons, which include cyclopropyl, cyclobutyl, cyclopentyl, cyclohexyl, cycloheptyl, cyclooctyl, cyclodecyl and cyclododecyl, any of which groups may be substituted with substituents such as halogen, lower alkyl, alkoxy and/or hydroxy groups.

The term "aryl" or "Ar" as employed herein by itself or as part of another group refers to monocyclic or bicyclic aromatic groups containing from 6 to l0 carbons in the ring portion, such as phenyl, or naphthyl. Aryl (or Ar), phenyl or naphthyl may include substituted aryl, substituted phenyl or substituted naphthyl, which may include l or 2 substituents on either the Ar, phenyl or naphthyl such as lower alkyl, cyano, amino, alkylamino, dialkylamino, nitro, carboxy, carboalkoxy, trifluoromethyl, halogen (Cl, Br, I or F), lower alkoxy, arylalkoxy, hydroxy, alkylthio, alkylsulfinyl, alkylsulfonyl, arylthio, arylsulfinyl and/or arylsulfonyl.

The term "aralkyl", "aryl-alkyl" or "aryl-lower alkyl" as used herein by itself or as part of another group refers to lower alkyl groups as discussed above having an aryl substituent, such as benzyl.

The term "lower alkoxy", "alkoxy" or aralkoxy" includes any of the above lower alkyl, alkyl or aralkyl groups linked to an oxygen atom.

The term "halogen" or "halo" as used herein by itself or as part of another group refers to chlorine, bromine, fluorine or iodine with chlorine being preferred.

The term "lower alkenyl" or "alkenyl" as employed herein by itself or as part of another group includes a carbon chain of up to l6 carbons, preferably 3 to l0 carbons, containing one double bond which will be separated from "N" by at least one saturated carbon moiety such as $-(CH_2)_q-$ where q can be l to l4, such as 2-propenyl, 2-butenyl, 3-butenyl, 2-pentenyl, 4-pentenyl and the like, and may include a halogen substituent such as I, Cl, or F.

The term "lower alkynyl" or "alkynyl" as employed herein by itself or as part of another group includes a carbon chain of up to l6 carbons, preferably 3 to l0 carbons, containing one triple bond which will be separated from "N" by at least one saturated carbon moiety such as $-(CH2)_{q'}-$ where q' can be l to l4, such as 2-propynyl, 2-butynyl, 3-butynyl and the like.

The term "heteroaryl" or heteroaromatic by itself or as part of another group refers to a 5- or 6-membered aromatic ring which includes l or 2 hetero atoms such as nitrogen, oxygen or sulfur, such as

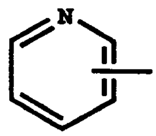 , 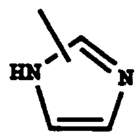 , 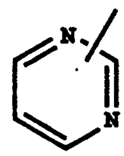 , 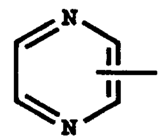 ,

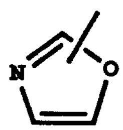 , 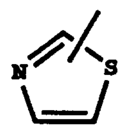 , 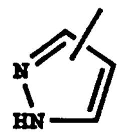 , 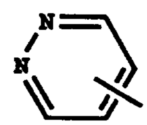 ,

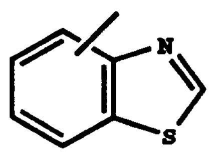 , 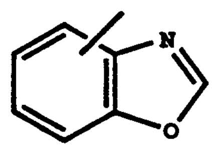 , 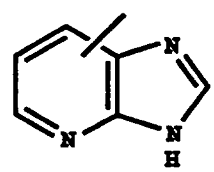 ,

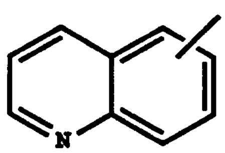 , 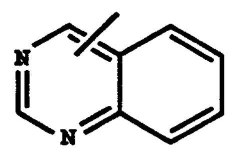 , 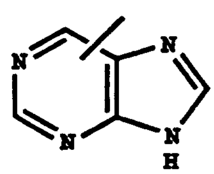

and the like. The heteroaryl rings may optionally be fused to aryl rings defined previously. The heteroaryl rings may optionally include l or 2 substituents such as halogen (Cl, Br, F or $CF_3$), lower alkyl, lower alkoxy, carboxy, amino, lower alkylamino and/or dilower alkylamino.

The term "amino acid side chain" refers to any of the known alpha-amino acids such as arginine, histidine, alanine, glycine, lysine, glutamine, leucine, valine, serine, homoserine, allothreonine, naphthylalanine, isoleucine, phenylalanine and the like.

Preferred are compounds of formula I wherein G is

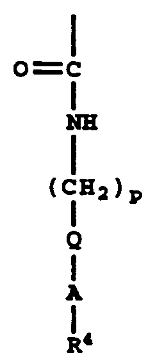

wherein Q is a single bond, A is an azacycloalkyl ring

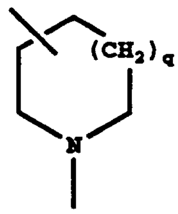

;

where q is 0 or l; and

$R^4$ is amidino;

$R^3$ is lower alkyl or aryl;

R is aralkyl or hydroxyalkyl;

$R^1$ and $R^2$ are each H;

n is 0 or l.

A more preferred embodiment of the heterocyclic thrombin inhibitors of the invention has the structure IA

IA

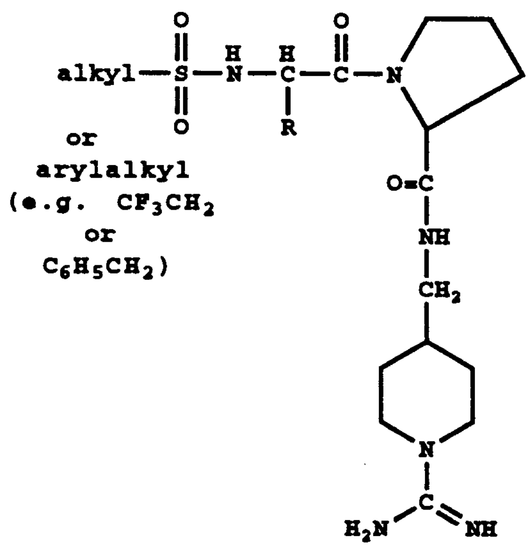

where R is aralkyl (preferably benzyl), aryl (preferably phenyl), or arylalkoryalkyl (preferably benzyloxymethyl) and alkyl is preferably methyl, ethyl or propyl, including all stereoisomers thereof.

Other preferred compounds of formula I are those wherein G is Gl, n is 0 or l; m is 2; $R^3$ is aryl or alkyl; R is arylalkyl or hydroxyalkyl such as hydroxymethyl; $R^1$ is hydrogen or lower alkyl such as methyl or ethyl; $R^2$ is H; and Y is -NH-; and compounds of formula IB:

IB

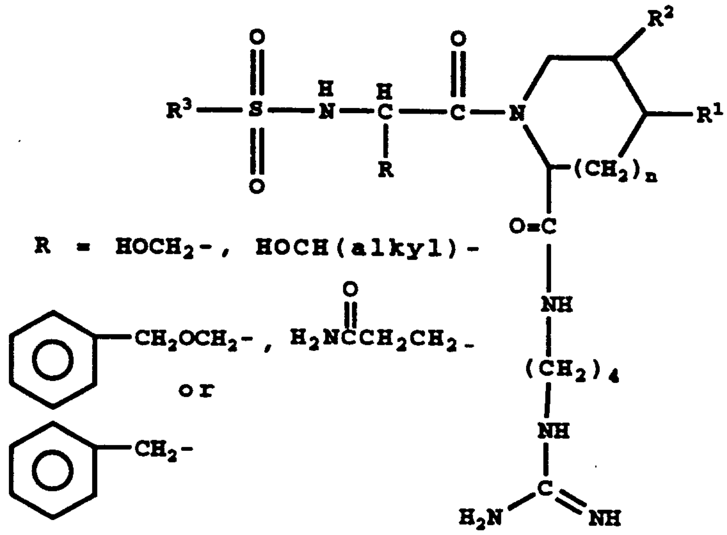

R = $HOCH_2$-, HOCH(alkyl)-

or

$R^3$=aryl, alkyl or arylalkyl

The compounds of formula I of the invention wherein G is

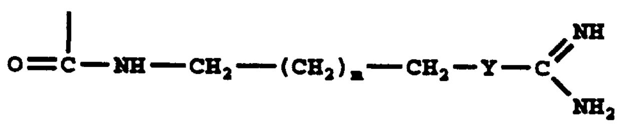

and Y is NH may be prepared according to the following Reaction Sequence I.

Reaction Sequence I

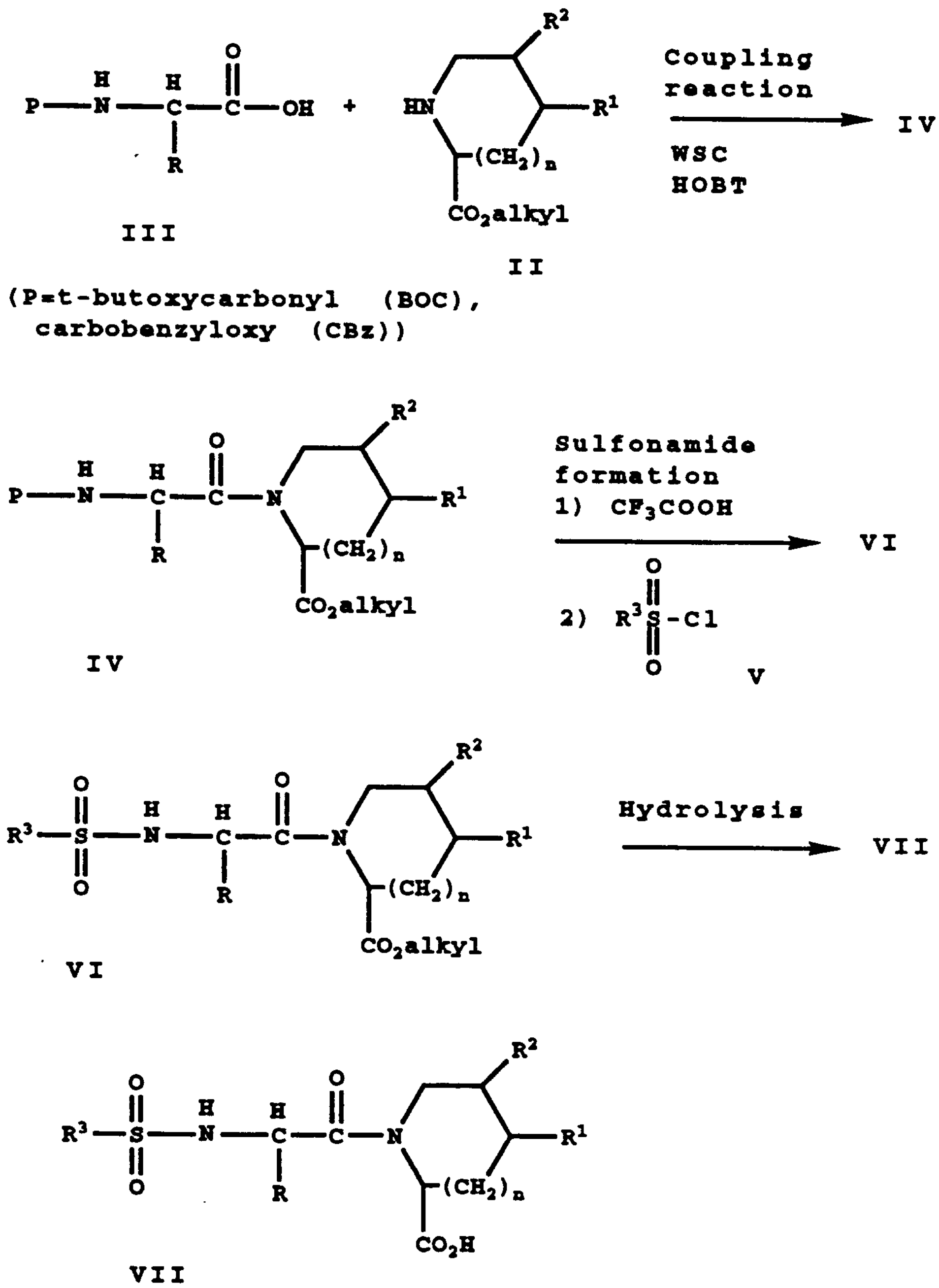


III

II

(P=t-butoxycarbonyl (BOC), carbobenzyloxy (CBz))

IV

V

VI

VII

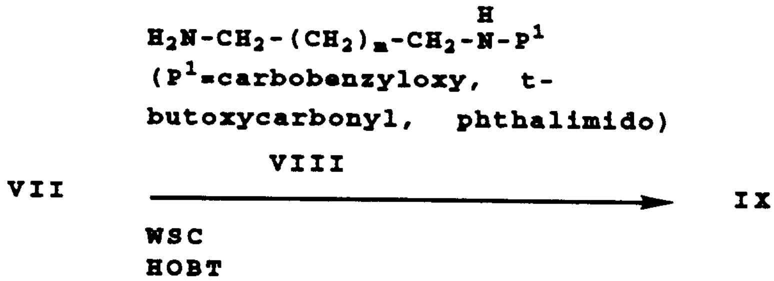


VII → IX

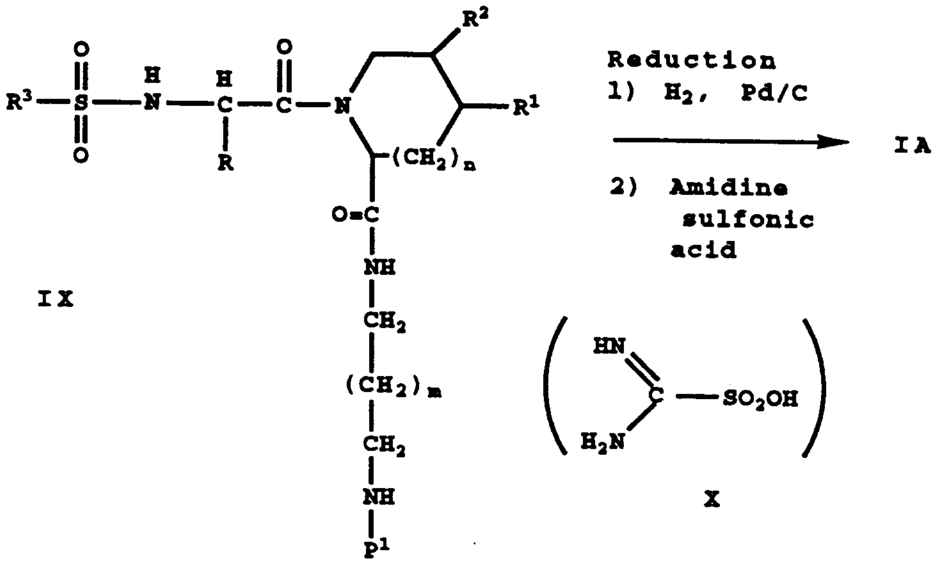


IX

IA

X

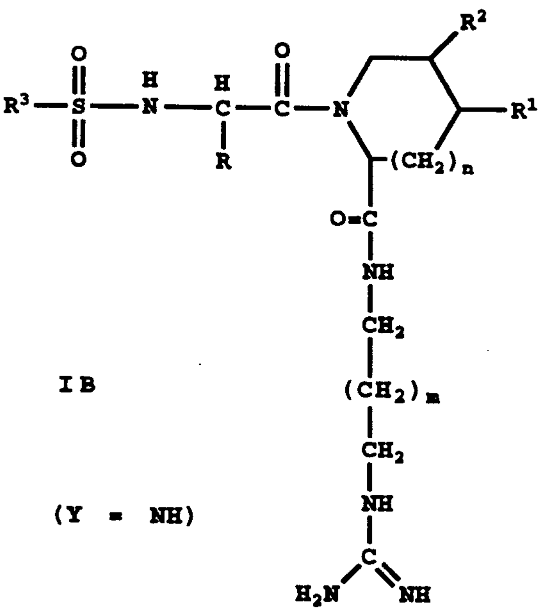

IB

(Y = NH)

The compounds of formula I of the invention wherein G is

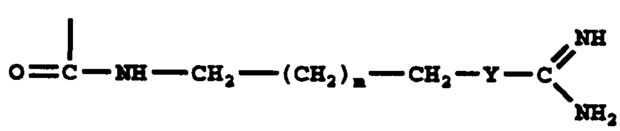

and Y is NH may also be prepared according to the following Reaction Sequence II

Reaction Sequence II

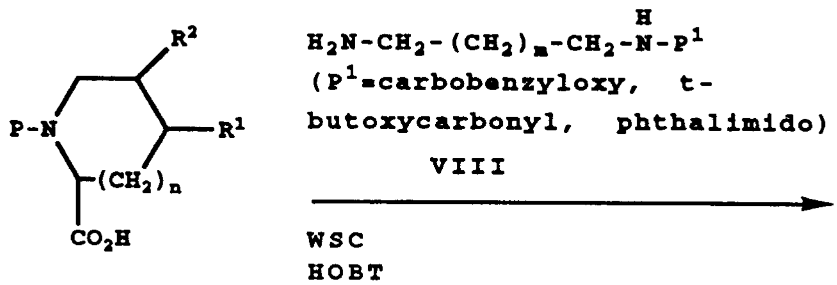

IXA

IIA

IXA

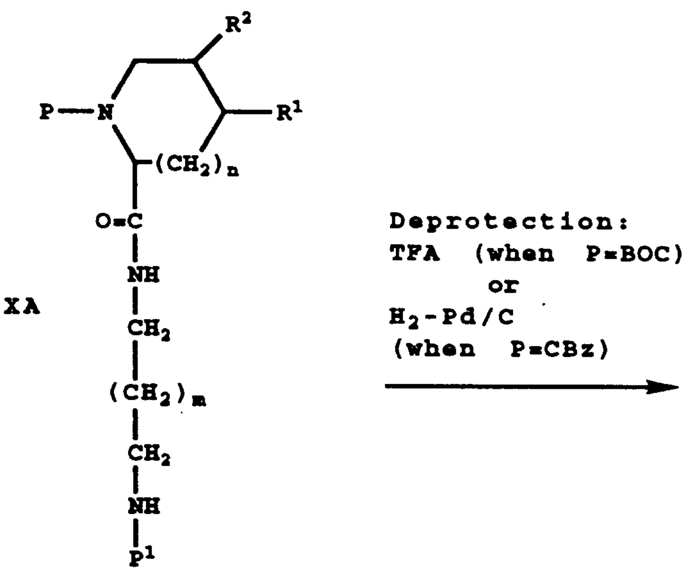

IXB

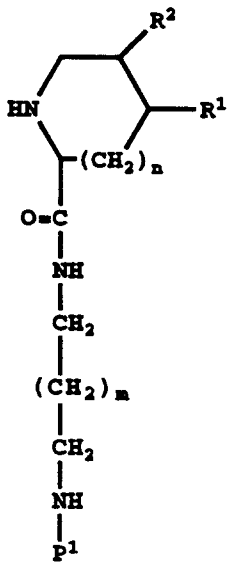

IXB

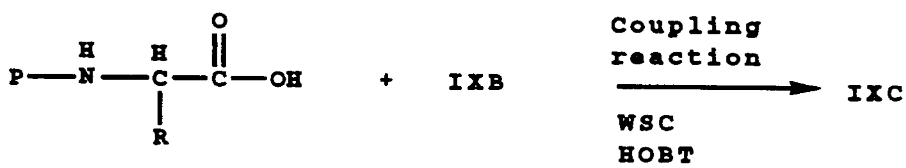


III

(P=BOC or CBz)

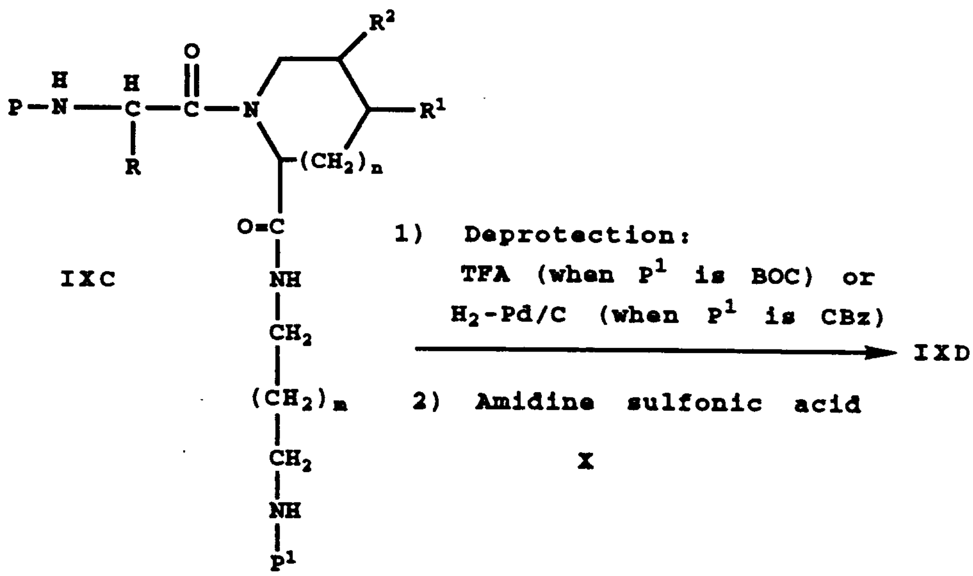

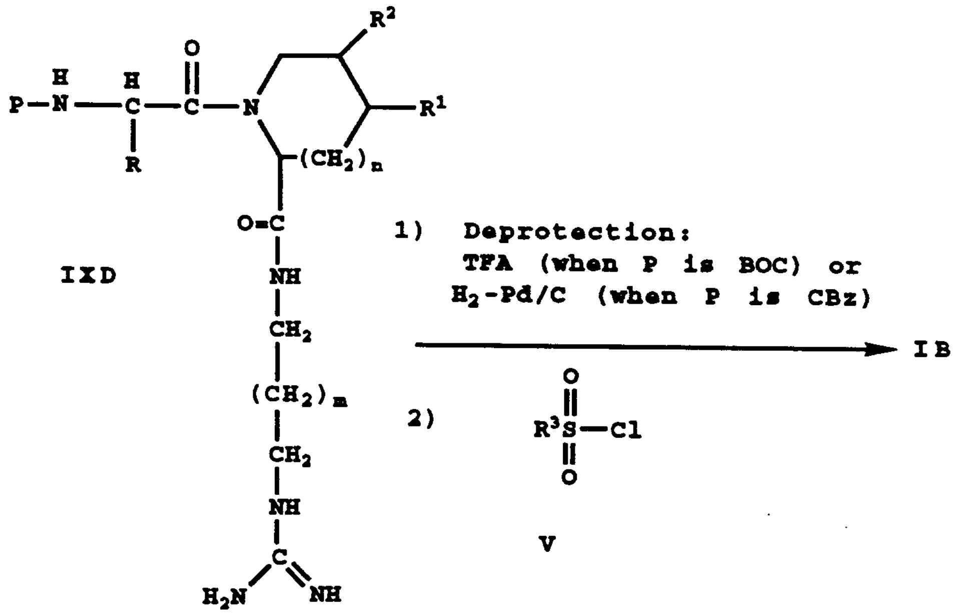


As seen in the above Reaction Sequence I, compounds of formula I wherein Y is -NH-, are prepared as follows. The ester II is made to undergo a carbodiimide coupling reaction with protected amino acid III in the presence of ethyl 3-(3-dimethylamino)propyl carbodiimide hydrochloride (WSC) or dicyclohexylcarbodiimide (DCC), and l-hydroxybenzotriazole monohydrate (HOBT), and N-methylmorpholine (NMM), and in the presence of an inert organic solvent such as dimethylformamide (DMF), THF or N-methylpyrrolidone, to form the amide IV. Amide IV is deprotected by treatment with trifluoroacetic acid with or without the presence of dry inert organic solvent such as dichloromethane, chloroform or THF at temperatures within the range of from about -l5° to about 20°C. Sulfonyl chloride V is added followed by organic base such as triethylamine, pyridine or N,N-diisopropylethylamine to form the sulfonamide VI. Sulfonamide VI is hydrolyzed by treatment with alkali metal base such as NaOH or LiOH in the presence of an alcohol solvent such as methanol or ethanol. The reaction mixture is acidified with HCl, $KHSO_4$ or $H_2SO_4$, to form acid VII. The acid VII is then subjected to a carbodiimide coupling reaction wherein VII is treated with protected amine VIII in the presence of WSC or DCC, and HOBT, and NMM, in the presence of an inert organic solvent such as dimethylformamide, THF or N-methylpyrrolidone, to form sulfonamide IX. The sulfonamide IX is then dissolved in an alcohol solvent such as ethanol or methanol, to which HCl has been added and the mixture is hydrogenated over Pd-C or $Pd(OH)_2$-C in the case where $P^1$ is carbobenzyloxy. The crude material is separated by conventional procedures and the desired isomers are treated with amidine sulfonic acid X in the presence of an alcohol solvent such as ethanol to form the compound of the invention IB.

As seen in the above Reaction Sequence II, compounds of formula I wherein Y is -NH-, are prepared as follows. The protected acid IIA is made to undergo a carbodiimide coupling reaction with protected amino acid VIII in the presence of ethyl 3-(3-dimethylamino)propyl carbodiimide hydrochloride (WSC) or dicyclohexylcarbodiimide (DDC), and l-hydroxybenzotriazole monohydrate (HOBT), and N-methylmorpholine (NMM), and in the presence of an inert organic solvent such as dimethylformamide (DMF), THF or N-methylpyrrolidone, to form the amide IXA. Amide IXA is deprotected by treatment with trifluoroacetic acid (TFA) when P is t-butoxycarbonyl (BOC) or $H_2$-Pd/C when P is carbobenzyloxy (CBz), with or without the presence of dry inert organic solvent such as dichloromethane, chloroform or THF, at temperatures within the range of from about -l5° to about 20°C to form amide IXB. The amide IXB is then subjected to a carbodiimide coupling reaction wherein IXB is treated with protected amine III in the presence of WSC or DCC, and HOBT, and NMM, in the presence of an inert organic solvent such as dimethylformamide, THF or N-methylpyrrolidone, to form amide IXC. The amide IXC is then dissolved in an alcohol solvent such as ethanol or methanol, to which HCl has been added and the mixture is hydrogenated over Pd-C or $Pd(OH)_2$-C in the case where $P^1$ is CBz or treated with trifluoroacetic acid when $P^1$ is BOC. The crude material is separated by conventional procedures and the desired isomers are treated with amidine sulfonic acid X in the presence of an alcohol solvent such as ethanol to form IXD. Compound IXD is then deprotected by treatment with TFA when P is BOC or by treatment with $H_2$-Pd/C when P is CBz, as described above, and sulfonyl chloride V is added followed by organic base such as triethylamine, pyridine or N,N-diisopropylethylamine to form the sulfonamide IB.

The compounds of formula I of the invention wherein G is

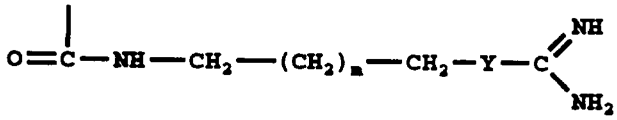

and Y is S may be prepared according to the following Reaction Sequence III.

Reaction Sequence III

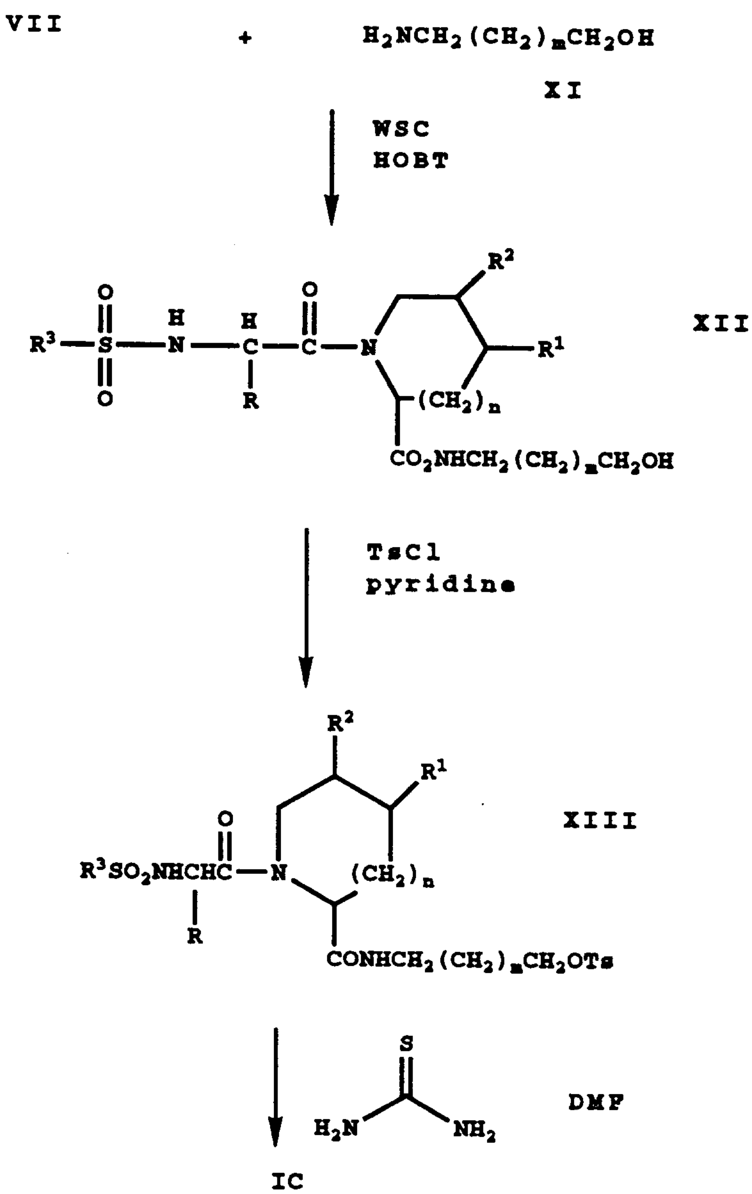

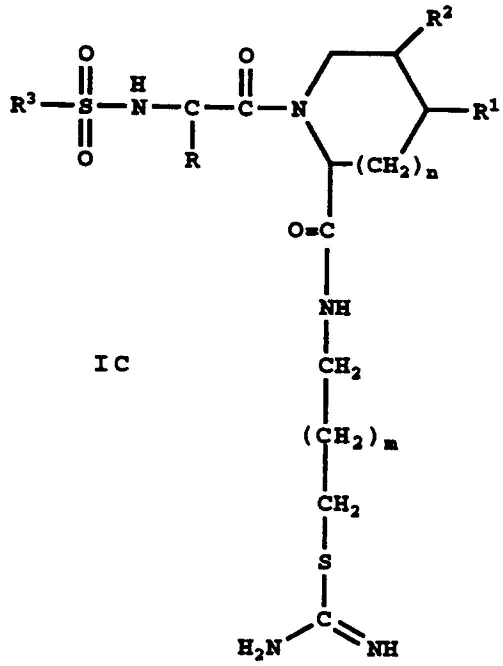

IC

Referring to the above Reaction Sequence III, compounds of formula I wherein Y = S can be prepared as follows. The acid VII is subjected to a carbodiimide coupling reaction wherein VII is treated with an aminoalcohol XI in the presence of WSC or DCC, HOBT, and NMM, in the presence of an inert organic solvent such as dimethylformamide, THF or N-methylpyrrolidone, to form sulfonamide alcohol XII. The sulfonamide alcohol XII is reacted with p-toluenesulfonyl chloride (TsCl) in pyridine, or in a solvent such as methylene chloride or chloroform, with N.H-dimethylaminopyridine to provide toluenesulfonate XIII. The compound IC (Y = S) is prepared by treating XIII with thiourea in a solvent such as DMF or DMSO at temperatures within the range of from about 25 ° C to about l00 ° C.

The compounds of formulae I and IA of the invention wherein G is

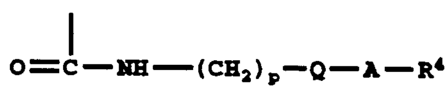

wherein A is azacycloalkyl or azaheteroalkyl, and $R^4$ is amidine, may be prepared according to the following Reaction Sequence IV:

Reaction Sequence IV

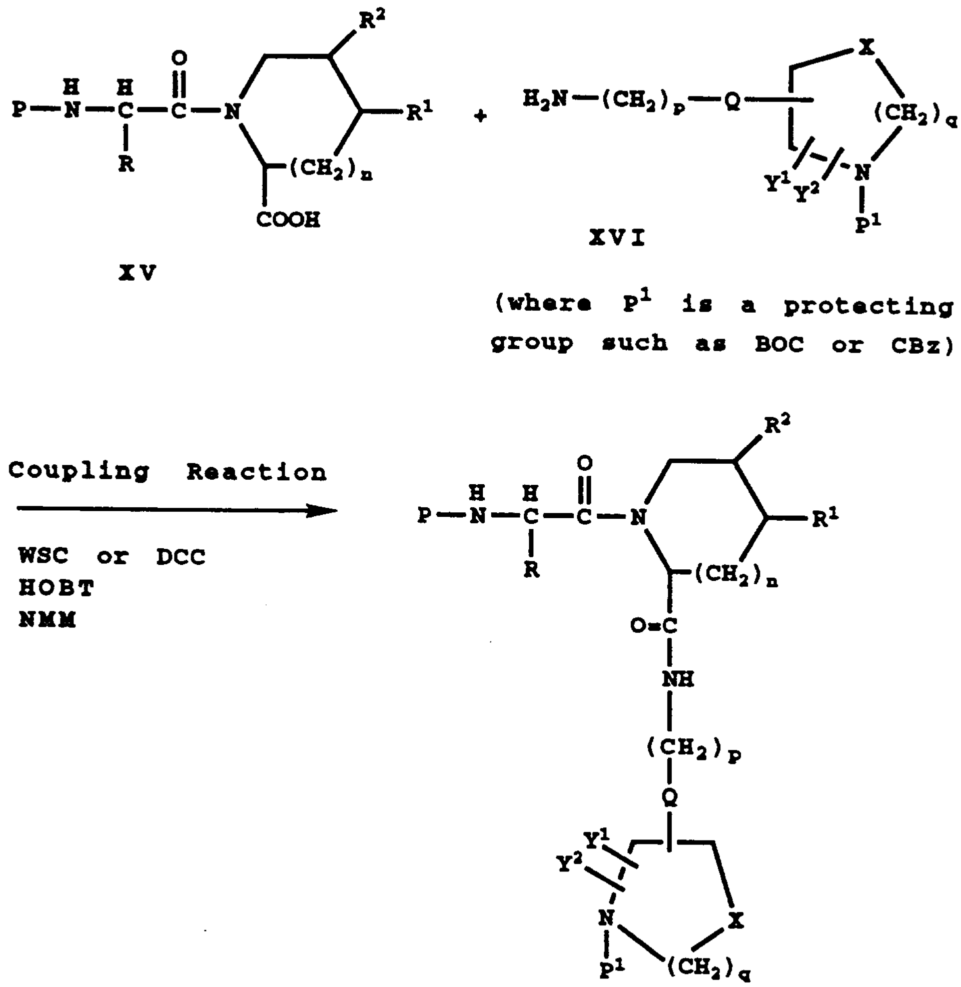


XV

XVI

(where $P^1$ is a protecting group such as BOC or CBz)

XVII

XVII

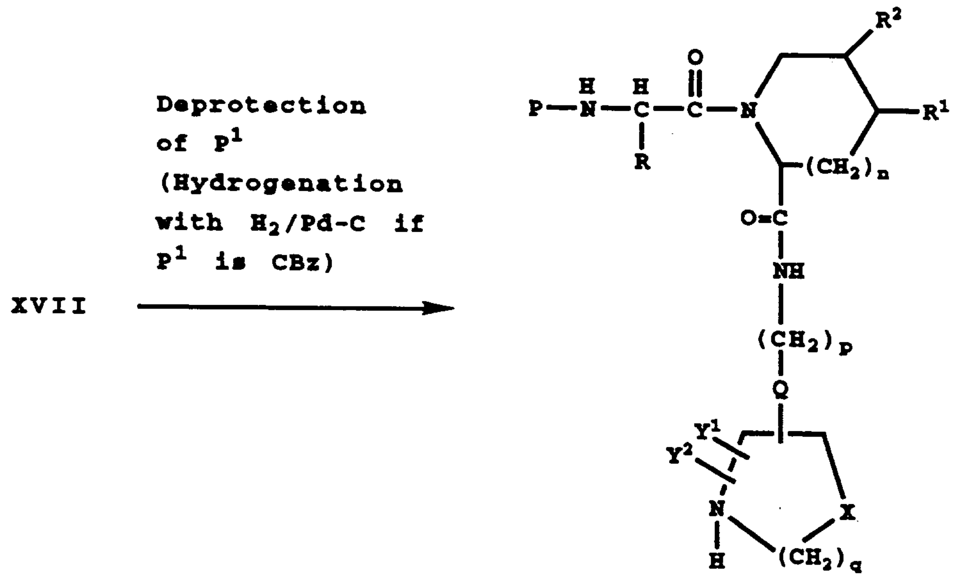


XVIII

XVIII

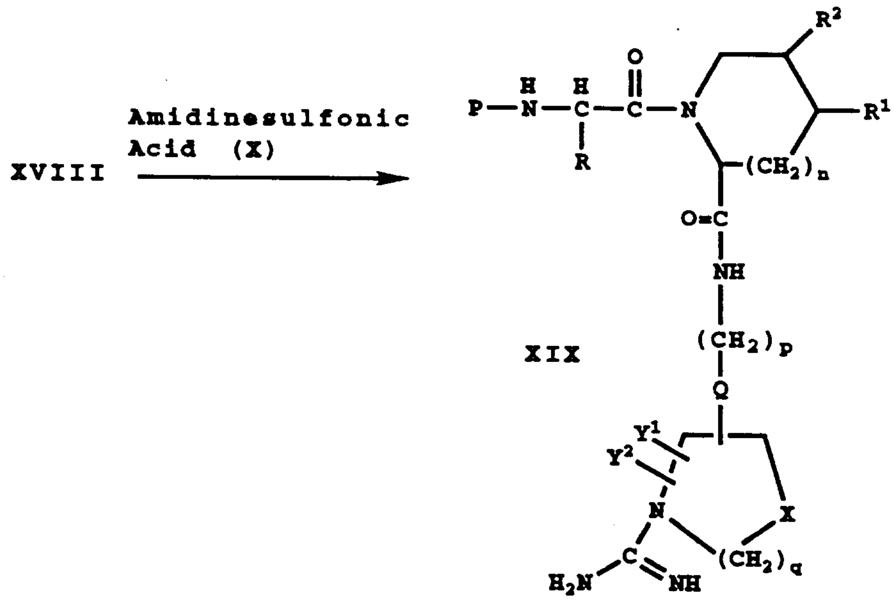


XIX

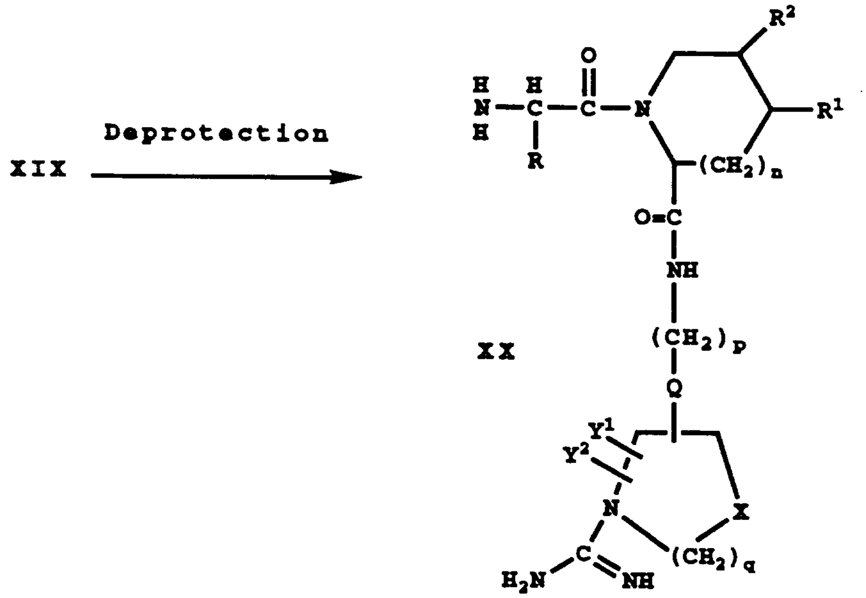


XX

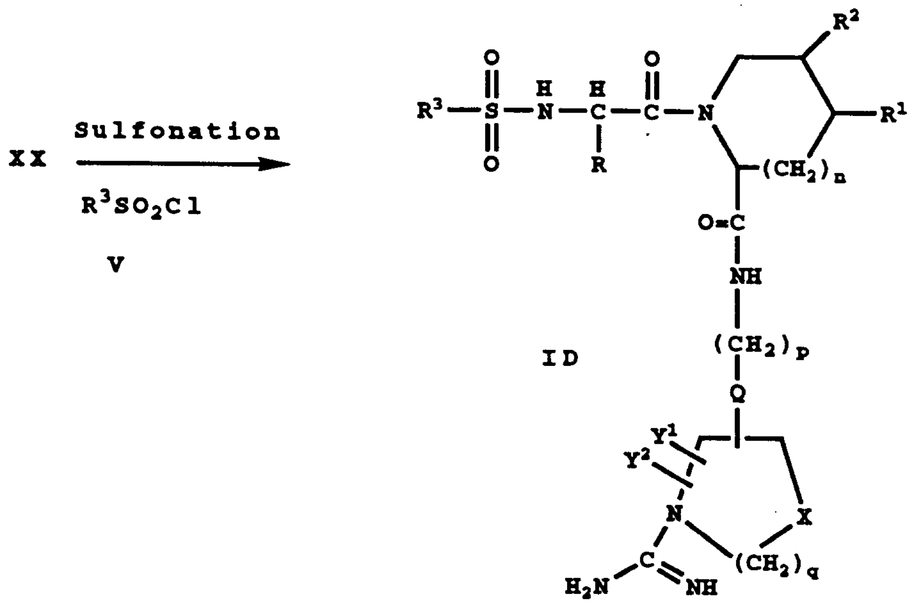


ID

As seen in the above Reaction Sequence IV, compounds of formula I wherein G is

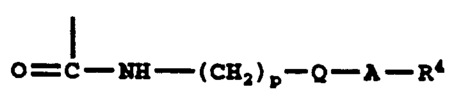

and A is azacycloalkyl or azaheteroalkyl, are prepared as follows. The protected acid XV is made to undergo a carbodiimide coupling reaction with amine XVI in the presence of ethyl 3-(3-dimethylamino)-propyl carbodiimide hydrochloride (WSC) or dicyclohexylcarbodiimide (DCC), and l-hydroxybenzotriazole monohydrate (HOBT), and N-methylmorpholine (NMM), and in the presence of an inert organic solvent such as dimethylformamide (DMF), THF or N-methylpyrrolidone, to form the amide XVII. Amide XVII is deprotected by treatment with, for example, $H_2$/Pd-C, if $P^1$ is CBz, to form amine XVIII. Amine XVIII is treated with amidinesulfonic acid X in the presence of alcohol solvent, such as ethanol to form amine XIX. Amine XIX is deprotected by treatment with trifluoroacetic acid (if P = BOC), with or without the presence of dry inert organic solvent such as dichloromethane, chloroform or THF, at temperatures within the range of from about -l5° to about 20°C. Sulfonyl chloride V is added followed by organic base such as

triethylamine, pyridine or N,N-diisopropylethylamine to form the sulfonamide ID of the invention.

The starting materials of formula XVI are known in the art or may be prepared by those skilled in the art employing conventional techniques.

The compounds of formulae I and IA of the invention where G is

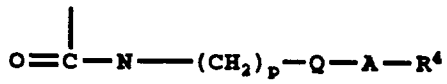

where A is aryl or cycloalkyl and $R^4$ is amidine or guanidine may be prepared according to the following Reaction Sequence V:

Reaction Sequence V

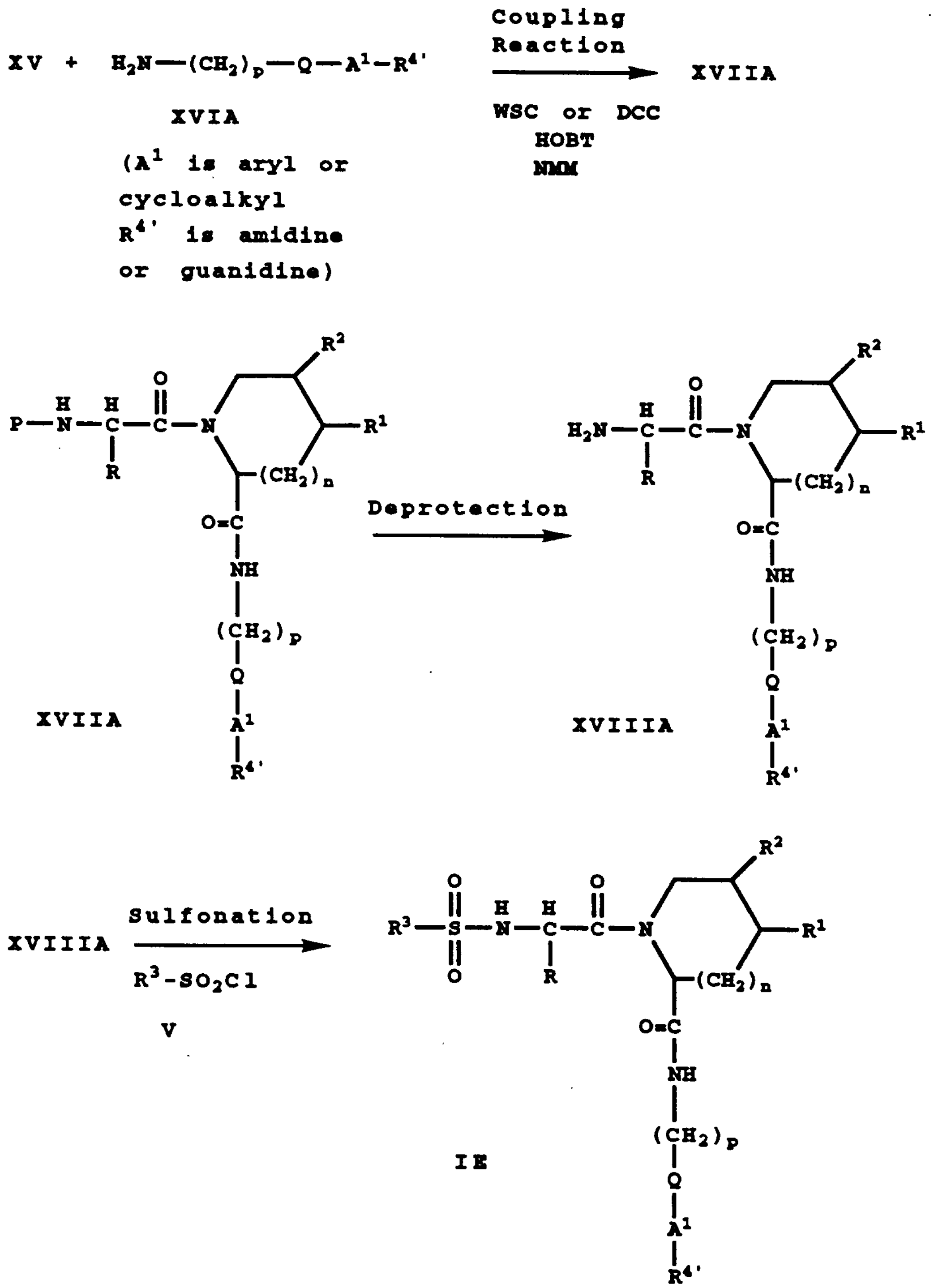


XVIA

XVIIA

XVIIIA

V

IE

As seen in Reaction Sequence V, compounds of formulae I and IA where G is

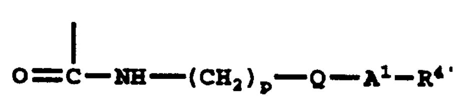

are prepared as follows. The protected acid XV is subjected to a carbodiimide coupling reaction wherein XV is treated with protected amine XVIA in the presence of WSC or DCC, and HOBT, and NMM, in the presence of an inert organic solvent such as dimethylformamide, THF or N-methylpyrrolidone, to form amide XVIIA. The amide XVIIA is then dissolved in an alcohol solvent such as ethanol or methanol, to which HCl has been added and the mixture is hydrogenated over Pd-C or $Pd(OH)_2$-C in the case where P is carbobenzyloxy. The amide XVIIIA is treated with sulfonyl chloride V followed by base to form the compound of the invention IE.

The starting compound XVIA is known in the art or may be prepared employing conventional procedures.

The compounds of formulae I and IA of the invention wherein G is

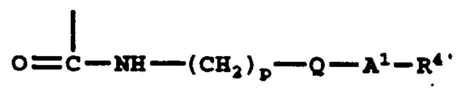

where A is aryl or cycloalkyl (that is $A^1$) and $R^4$ is aminomethyl (that is $R^{4''}$) may be prepared according to the following Reaction Sequence VI:

Reaction Sequence VI

XV + $H_2N-(CH_2)_p-Q-A^1-CH_2NHP^1$

XVIB

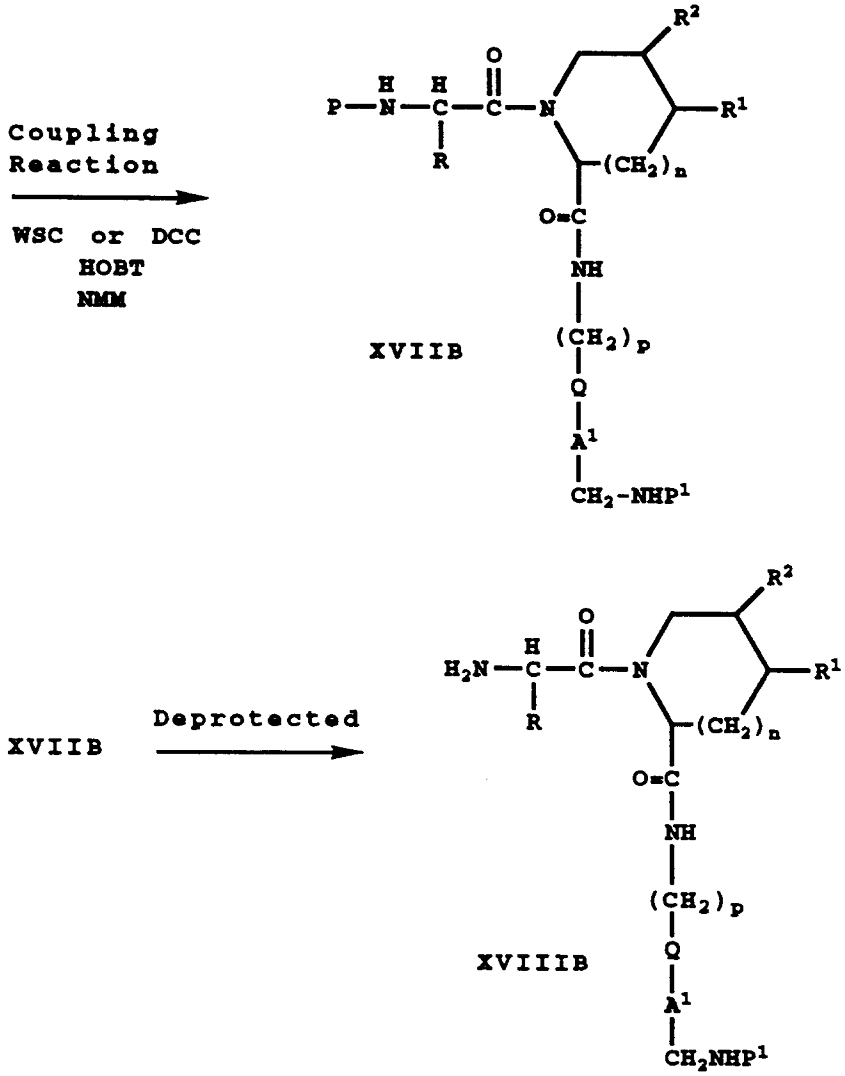


XVIIB

XVIIB

XVIIIB

XVIIIB

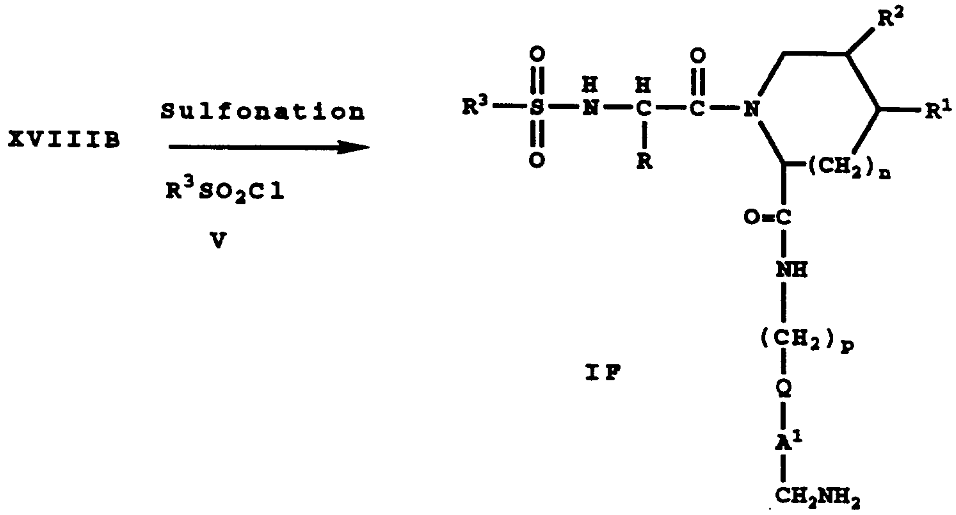


IF

As seen in the above Reaction Sequence VI, compounds of formulae I and IA wherein G is

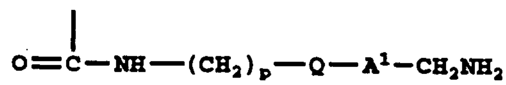

are prepared as follows. The protected acid XV is made to undergo a carbodiimide coupling reaction with protected amino acid XVIB in the presence of ethyl 3-(3-dimethylamino)propyl carbodiimide hydrochloride (WSC) or dicyclohexylcarbodiimide (DCC), and l-hydroxybenzotriazole monohydrate (HOBT), and N-methylmorpholine (NMM), and in the presence of an inert organic solvent such as dimethylformamide (DMF), THF or N-methylpyrrolidone, to form the amide XVIIB. Amide XVIIB is deprotected by treatment with trifluoroacetic acid (TFA) when P is t-butoxycarbonyl (BOC) or $H_2$-Pd/C when P is carbobenzyloxy (CBz), with or without the presence of dry inert organic solvent such as dichloromethane, chloroform or THF, at temperatures within the range of from about -l5° to about 20°C to form amide XVIIIB. The amide XVIIIB is then subjected to a sulfonation reaction wherein amide XVIIIB is reacted with sulfonyl chloride V in the presence of organic base such as triethylamine, pyridine or N,N-diisopropylethylamine to form the sulfonamide IF of the invention.

The starting compounds XVIB are known in the art or may be prepared employing conventional procedures.

The starting acid XV may be prepared from ester IV by hydrolyzing ester IV by treating with a base such as NaOH, KOH or LiOH and then neutralizing the resulting alkali metal salt with strong acid such as HCl or oxalic acid.

The compounds of formula I of the invention can be obtained as pharmaceutically acceptable acid addition salts by reacting a free base with an acid, such as hydrochloric, hydrobromic, hydroiodic, nitric, sulfuric, phosphoric, acetic, citric, maleic, succinic, lactic, tartaric, gluconic, benzoic, methanesulfonic, ethanesulfonic, benzenesulfonic, p-toluenesulfonic acid or the like.

The compounds of the present invention are serine protease inhibitors, and in particular may inhibit thrombin, Factor Xa, and/or trypsin. The compounds of the present invention are useful for the treatment or prophylaxis of those processes which involve the production and/or action of thrombin. This includes a number of thrombotic and prothrombotic states in which the coagulation cascade is activated which include, but are not limited to, deep vein thrombosis (DVT), disseminated intravascular coagulopathy (DIC), Kasabach-Merritt syndrome, pulmonary embolism, myocardial infarction, stroke, thromboembolic complications of surgery (such as hip replacement and endarterectomy) and peripheral arterial occlusion. In addition to its effects on the coagulation process, thrombin has been shown to activate a large number of cells (such as neutrophils, fibroblasts, endothelial cells, smooth muscle cells). Therefore, the compounds of the present invention may also be useful for the treatment or prophylaxis of adult respiratory distress syndrome, septic shock, septicemia, inflammatory responses which include, but are not limited to, edema, acute or chronic atherosclerosis, and reperfusion damage.

The compounds of the invention may also be useful in treating neoplasia/metastasis (in particular those which utilize fibrin) and neurodegenerative diseases such as Alzheimer's disease and Parkinson's disease. In addition, the compounds of the present invention may be useful to prevent restenosis following arterial injury induced by endogenous (rupture of an atherosclerotic plaque) or exogenous (invasive cardiological procedure) events.

The compounds of the present invention may also be used as an anticoagulant in extracorpeal blood circuits, such as those necessary in dialysis and surgery (such as coronary artery bypass surgery).

The compounds of the present invention may also be used in combination with thrombolytic agents, such as tissue plasminogen activator (natural or recombinant), streptokinse, urokinase, prourokinase, anisolated streptokinase plasminogen activator complex (ASPAC), animal salivary gland plasminogen activators, and the like. The compounds of the present invention may act in a synergistic fashion to prevent reocclusion following a successful thrombolytic therapy and/or reduce the time to reperfusion. The compounds of the present invention may also allow for reduced doses of the thrombolytic agent to be used and therefore minimize potential hemorrhagic side-effects.

The compounds of the present invention may also be used in combination with other antithrombotic or anticoagulant drugs such as thromboxane receptor antagonists, prostacyclin mimetics, phosphodiesterase inhibitors, fibrinogen antagonists, and the like.

Compounds of the present invention that inhibit trypsin may also be useful for the treatment of pancreatitis.

The compounds of the invention can be administered orally or parenterally to various mammalian species known to be subject to such maladies, e.g., humans, cats, dogs and the like in an effective amount within the dosage range of about 0.l to about l00 mg/kg, preferably about 0.2 to about 50 mg/kg and more preferably about 0.5 to about 25 mg/kg (or from about l to about 2500 mg, preferably from about 5 to about 2000 mg) on a regimen in single or 2 to 4 divided daily doses.

The active substance can be utilized in a composition such as tablet, capsule, solution or suspension containing about 5 to about 500 mg per unit of dosage of a compound or mixture of compounds of formula I. They may be compounded in conventional matter with a physiologically acceptable vehicle or carrier, excipient, binder, preservative, stabilizer, flavor, etc., as called for by accepted pharmaceutical practice.

The following Examples represent preferred embodiments of the present invention. Unless otherwise indicated, all temperatures are expressed in degrees Centigrade.

Example I

[l(S),2α,4β]-N-[4-[(Aminoiminomethyl)amino]butyl]-l-[3-hydroxy-2-[(2-naphthalenylsulfonyl)amino]-l-oxoprpoyl]-4-methyl-2-piperidinecarboxamie, trifluoroacetate (l:l) salt

A. (2R-trans)-l-[(Phenylmethoxy)carbonyl]-4-methyl-2-piperidinecarboxylic acid, ethyl ester

A(l). 4-Methyl-2-piperidinecarbonitrile

To 500 g of sodium hypochlorite solution (5% in Cl, Aldrich) cooled in an ice bath was added dropwise 33.6 g (340 mmol, Aldrich) of 4-methyl-piperidine, over 40 minutes. The reaction mixture was stirred for 20 minutes then poured into a separatory funnel and extracted with two-400 mL portions of ether. The extracts were combined, dried (sodium sulfate) and concentrated in vacuo to give ~44 g of N-chloro intermediate as a yellow liquid.

To a solution of l9.l g (340 mmol) of potassium hydroxide in l40 mL of 95% ethanol heated to 80° was added dropwise over 40 minutes the solution of crude N-chloropiperidine from above in 20 mL of 95% ethanol. The addition was mildly exothermic and a precipitate formed. The reaction mixture was stirred for l0 minutes, cooled to room temperature, concentrated in vacuo, and to the residue was added 85 mL of 2N aqueous NaOH solution. The resulting mixture was extracted with three 75 mL portions of ether. The ether extracts were combined, dried (sodium sulfate) and concentrated in vacuo to give the crude imine as a viscous yellow oil.

To a solution of ll0 g (l.7 mol, Mallinckrodt) of potassium cyanide in 400 mL of water cooled in an ice-bath was added over ~l h l83 mL (2.20 mol) of concentrated HCl, followed by the crude imine from above. The reaction mixture was stirred between l0-20°C for 4 h then cooled in an ice-bath and basified to pH l2 by addition of ~80 g of potassium hydroxide pellets. the resulting solution was poured into a separatory funnel and extracted with three-300 mL portions of ether. The ether layers were combined, dried (sodium sulfate) and concentrated in vacuo to give a yellow oil. The crude material was purified by simple distillation

at reduced pressure to afford l3.2 g (l06 mmol, 3l%) of title nitrile as a clear liquid, bp 72-74° (3mm).

A(2). 4-Methyl-2-piperidinecarboxylic acid, ethyl ester

To 250 mL of 6N aqueous HCl solution was added l3.0 g (l05 mmol) of Part A(l) nitrile at room temperature. The reaction mixture was heated to reflux (bath temp l45°) for 6 h then cooled to room temperature and concentrated in vacuo to give the crude acid hydrochloride as a solid.

To. 250 mL of absolute ethanol cooled in an ice-bath was added dropwise 40 mL (550 mmol) of thionyl chloride over 30 minutes. The solution was stirred for an additional l5 minutes then added to the crude acid hydrochloride from above. The resulting slurry was heated to reflux for 4 h then cooled to room temperature and filtered to remove solids. The filtrate was concentrated in vacuo to give the crude ester amine hydrochloride as brown oil. The oil was partitioned between l50 mL of saturated aqueous potassium carbonate solution and l50 mL of chloroform. The aqueous layer was separated and extracted with two-l00 mL portions of chloroform. The organic extracts were combined, dried (sodium sulfate) and concentrated to give crude title ester as a brown oil. The crude material was purified by distillation through a l0 cm packed column (glass helices, 3mm) at reduced pressure to afford ll.0 g (64.3 mmol, 6l%) of title amine as a colorless liquid, bp 37-38° (0.4mm). The trans/cis ratio was determined as ~6:l by 270.MHz $^1$H NMR.

A(3). (2R-trans)-l-[(Phenylmethoxy)-carbonyl]-4-methyl-2-piperidinecarboxylic acid, ethyl ester

To a solution of l0.0 g (58.5 mmol) of Part A(2) amine in l00 mL of methylene chloride cooled to 0° was added 9.8 mL (70 mmol, distilled from calcium hydride) of triethylamine in one portion then dropwise ll.9 g (70 mmol, Aldrich) of benzyl chloroformate over 20 minutes. The reaction mixture was stirred for 30 minutes then washed with l00 mL of lN aqueous HCl, l00 mL of saturated aqueous sodium bicarbonate solution, 50 mL of brine, dried (magnesium sulfate) and then concentrated in vacuo to give an oil. The crude oil was purified by flash chromatography (Merck silica, 30xl0cm, 600 g, l:9 EtOAc/hexane) to afford l2.7 g (4l.6 mmol, 7l%) of title compound (trans isomer) as a colorless oil. In addition 2.95 g (9.67 mmol, l7%) of a ~l:l mixture of trans/cis isomers was obtained as a colorless oil.

B. (2R-trans)-4-Methylpiperidinecarboxylic acid, ethyl ester

Part A racemic CBZ ester (2.36 g, 7.73 mmol) was dissolved in absolute ethanol (50 mL) and hydrogenated over Pd-C (10%, 250 mg) at RT and 1 atm. After 6 hrs additional catalyst (50 mg) was added, the reaction continued for 2 hours, filtered, and evaporated in vacuo to provide the title free amine (1.22 g, 7.13 mmol, 92%).

C. (2R-trans)-l-[N-[(l,l-Dimethylethoxy)-carbonyl]-O-(phenylmethyl)-L-seryl]-4-methyl-2-piperidinecarboxylic acid, ethyl ester

Part B pipecolic ester (1.22 g, 7.13 mmol) and BOC-Ser(OBn)-OH (2.36 g, 8.0 mmol) were dissolved in DMF (15 mL) at RT. HOBT (1.08 g, 8.0 mmol), WSC (1.54 g, 8.0 mmol) and N-methyl morpholine (NMM) (0.88 mL, 8.0 mmol) were added. The pH was ca 8.5. The reaction was stirred for 90 min, partitioned between ethyl acetate and 10% $KHSO_4$, the aqueous layer back-extracted, and the organic layers combined. The combined organic layers were washed with saturated $NaHCO_3$ (2 X), saturated NaCl, dried over magnesium sulfate and stripped in vacuo to provide title ester as an oil (2.99 g, 6.67 mmol, 93%).

D. (2R-trans)-l-[N-(2-Naphthalenylsulfonyl)-O-(phenylmethyl)-L-seryl]-4-methyl-2-piperidinecarboxylic acid, ethyl ester

Part C BOC-Ser-pipecolic ester (2.80 g, 6.25 mmol) was dissolved in trifluoroacetic acid (TFA) at 0°C, and allowed to warm to RT over 60 min. The TFA was evaporated in vacuo for two hours to provide the TFA salt as an oil (4.2 g). The crude TFA salt was dissolved in dichloromethane (15 mL) and 2-naphthylsulfonyl chloride (Aldrich, 1.58 g, 7.0 mmol) was added, followed by triethylamine (3.5 mL, 25 mmol). A vigorous exotherm developed, and the reaction was put into an ice bath for 10 min, then warmed to RT. After 6 hr the dichloromethane was evaporated, the reaction mixture partitioned between ethyl acetate and 10% $KHSO_4$ (2 X), washed with saturated $NaHCO_3$ (2 X), dried over magnesium sulfate and evaporated to provide crude title ester (3.18 g). The crude product was chromatographed on silica gel (1:3 = => 1:2 ethyl acetate (EtOAc):hexanes to provide the two diastereomers as an oil (2.29 g, 4.25 mmol,

68% over two steps).

E. (2R-trans)-l-[N-(2-Naphthalenylsulfonyl)-O-(phenylmethyl)-L-seryl]4-methyl-2-piperidinecarboxylic acid

The Part D ester was dissolved in methanol (MeOH) (30 mL) and treated with 1N NaOH (10 mL, 10 mmol). The reaction was stirred for 12 hr, and additional NaOH (5 mL) was added. After 24 hr the reaction was acidified with HCl (50 mL, 1N), extracted with ethyl acetate (2 X), and dried in vacuo to give title free acid (1.99 g, 3.90 mmol, 99%).

F. (4-Aminobutyl)carbamic acid, phenylmethyl ester

1,4-butanediamine dihydrochloride (5.96 g, 37 mmol) was dissolved in water (15 mL), and DMF (50 mL) was added. With rapid stirring, CBZ-Cl (1.0 mL, 7.0 mmol) was added dropwise over 2 minutes. The cloudy solution had pH ~ 2.8. The pH was adjusted to 9.0 with 5 N NaOH, (clear solution) and stirred for 2 hr. The reaction mixture was acidified to pH = 1.25, extracted with ether (2 X), made basic (pH > 10) with 5 N NaOH, and extracted with dichloromethane (2 X). The dichloromethane fractions were combined and washed with water (2 X), dried and evaporated in vacuo to provide the crude mono-CBZ amine (0.72 g, 3.24 mmol, 46%) which was used as is in the next step.

G. (2R-trans)-l-[N-(2-Naphthalenylsulfonyl)-O-(phenylmethyl)-L-seryl]-N-[4-[[(phenylmethoxy)carbonyl]-amino]butyl]-4-methyl-2-piperidinecarboxamide

To a solution of Part E mono-CBZ amine (400 mg, 1.8 mmol) and Part E acid (640 mg, 1.25 mmol) in DMF (5 mL) was added HOBT (170 mg, 1.25 mmol), WSC (0.25 g, 1.3 mmol), and NMM (138 µL, 1.3 mmol). The pH was ~ 8.5. The reaction was stirred for two hr (complete by HPLC), partitioned between ethyl acetate and 10% $KHSO_4$, back-extracted, and the combined organic layers washed with 10% $KHSO_4$, saturated $NaHCO_3$ (2 X), saturated NaCl, and evaporated to provide the crude product in quantitative yield. The crude product was chromatographed on silica gel (2:1::EtOAc:hexanes) to provide a mixture of the title two diastereomers (671 mg, 0.94 mmol, 75%).

H. [l(S),2α,4β]-N-[4-[(Aminoiminomethyl)-amino]butyl]-l-[3-hydroxy-2-[(2-naphthalenylsulfonyl)amino]-l-oxopropyl]-4-methyl-2-piperidinecarboxamide, trifluoroacetate (l:l) salt

The Part G O-benzyl CBZ derivative was dissolved in ethanol (20 mL) to which acetyl chloride had been added (0.71 mL, 10 mmol, gives a 0.5 M solution of HCl), and the mixture hydrogenated over Pd-C (10%, 150 mg) at 1 atm and reflux temperature. After 5 hr the reaction mixture was filtered, stripped and fresh ethanol and catalyst added. The reaction was determined to be ca 80% complete after an additional 2 hr, whereupon it was filtered and stripped to provide the crude amine (412 mg). The crude material was purified by preparative HPLC (80:20 => 50:50) to provide the des-benzyl, des-CBZ diastereomers (281 mg, 0.57 mmol, 71%) and the O-benzyl, des-CBZ compounds (69 mg, 0.12 mmol, 15%).

The des-benzyl, des-CBZ material above (208 mg, 0.46 mmol) was dissolved in ethanol (5 mL), to which amidinesulfonic acid (78 mg, 0.65 mmol) and triethyl amine (176 µL, 1.26 mmol) were added. After 20 hr the solvent was evaporated and the material purified by preparative HPLC (80:20=>50:50). Fractions containing the two major components were combined and lyophilized to provide title compound as a 40:60 mixture of isomers A:B (255 mg, 78%). Purity ≧ 98%.

$[\alpha]_D$ (c = 1.0, MeOH) = -11.7°.

| Analysis calc'd for 1.30 TFA + 0.80 $H_2O$: | | | | |
|---|---|---|---|---|
| | C, 47.68; | H, 5.64; | N, 12.09; | F, 10.66. |
| Found: | C, 47.68; | H, 5.34;, | N, 11.97; | F, 10.83. |

Example 2

[1(S),2α,4β]-N-[4-[(Aminoiminomethyl)amino]butyl]-4-methyl-l-[2-[(2-naphthalenylsulfonyl)amino]-l-oxo-3-(phenylmethoxy)propyl]-2-piperidinecarboxamide, trifluoroacetate (l:l) salt

The O-benzyl, des-CBZ compound described in Example I Part H (69 mg, 0.099 mmol) was dissolved in ethanol (2 mL), to which amidinesulfonic acid (22 mg, 0.18 mmol, prepared as described in Synthesis (l986) 777-779), and triethyl amine (50 µL, 0.36 mmol) were added. After 20 hr the solvent was evaporated and the material purified by preparative HPLC (80:20=>50:50). Fractions containing the two major components were combined and lyophilized to provide title compound as a 1:3 mixture of isomers A:B (76 mg, 100%). Purity ≧ 98%.
$[\alpha]_D$ (c = 1.0, MeOH) = -5.6°.

| Analysis calc'd for 1.10 TFA + 0.30 $H_2O$: | | | | |
|---|---|---|---|---|
| | C, 54.51; | H, 5.84; | N, 11.15; | F, 8.32. |
| Found: | C, 54.19; | H, 5.84;, | N, 11.04; | F, 8.29. |

Example 3

[2R-[l(S*),2α,4β]-N-[4-[(Aminoiminomethyl)amino]butyl]-l-[3-hydroxy-2-[(2-naphthalenylsulfonyl)amino]-l-oxopropyl]-4-methyl-2-piperidinecarboxamide, trifluoroacetate (l:l) salt

Example I compound (ca 220 mg) was dissolved in water (15 mL) and chromatographed on a YMC S-10 20mm X 500 mm ODS column, using an 80:20 ==> 50:50 gradient. After lyophilization, the first peak to elute (isomer A) comprised 95 mg. The stereochemistry (2R, 4R) was assigned by comparison to a sample prepared form non-racemic starting material.
[α]D (c = 1.00, MeOH) + 11.0°.

| Analysis calc'd for 1.16 $H_2O$ + 1.07 TFA: | | | | | |
|---|---|---|---|---|---|
| | C, 48.25; | H, 5.88; | N, 12.44; | F, 9.03; | S, 4.75. |
| Found: | C, 48.25; | H, 5.61; | N, 12.25; | F, 9.25; | S, 5.19. |

Example 4

[2S-[l(R*),2α,4β]-N-[4-[(Aminoiminomethyl)amino]butyl]-l-[3-hydroxy-2-[(2-naphthalenylsulfonyl)amino]-l-oxopropyl]-4-methyl-2-piperidinecarboxamide, trifluoroacetate (l:l) salt

Example I compound (ca 220 mg) was dissolved in water (15 mL) and chromatographed on a YMC S-10 20mm X 500 mm ODS column, using an 80:20 ==> 50:50 gradient (acetonitrile:water). After lyophilization, the second peak to elute (isomer B) comprised 103 mg. The stereochemistry (2S, 4S) was assigned by comparison of the two isomers.
[α]D (c = 1.00, MeOH) -29.0°.

| Analysis calc'd for 1.39 $H_2O$ + 1.17 TFA: | | | | | |
|---|---|---|---|---|---|
| | C, 47.52; | H, 5.83; | N, 12.16; | F, 9.65; | S, 4.64. |
| Found: | C, 47.52; | H, 5.49; | N, 11.96; | F, 9.60; | S, 5.25. |

High resolution MS shows $(M+H)^+$ = 533.2546 ($C_{25}H_{37}O_5N_6S$).

Example 5

(2S-trans)-N-[4-[(Aminoiminomethyl)amino]butyl]-4-methyl-l-[[[(l,2,3,4-tetrahydro-3-methyl-8-quinolinyl)-sulfonyl]amino]acetyl]-2-piperidinecarboxamide, trifluoroacetate salt

A. (2S-trans)-4-Methyl-2-piperidinecarboxylic acid, ethyl ester

To a solution of dry EtOH (65 mL) and acetyl chloride (6.52 mL added dropwise at 0 °C) was added (2S, 4S)-4-methyl pipecolic acid (2.8 g, 19.6 mmol) at room temperature. The reaction was stirred at room temperature for 16 hrs and then concentrated in vacuo to give the title compound, (3.01 g, 90%).

B. (2S-trans)-l-[[[(l,l-Dimethylethoxy)carbonyl]amino]acetyl]-4-methyl-2-piperidinecarboxylic acid, ethyl ester

To a solution of L-Boc-Glycine (0.4 g, 2.25 mmol) and HOBT (0.31 g, 2.25 mmol) in 7 mL DMF was added WSC ( 0.44 g, 2.25 mmol) and Part A ethyl ester (0.35 g, 2.04 mmol), followed by NMM (240 µl, 2.25 mmol) to adjust the pH of the solution to 8. The reaction was stirred at room temperature for 16 hrs and worked up by washing with a saturated solution of $NaHCO_3$, a saturated solution of $KHSO_4$, brine, dried over $Na_2SO_4$ and evaporated to give the title compound (0.60 g, 89%).
$(M+H)^+$ @329

C. (2S-trans)-l-[[[(l,l-Dimethylethoxy)carbonyl]amino]acetyl]-4-methyl-2-piperidinecarboxylic acid

To a solution of Part B (560 mg, 1.71 mmol) in 6.5 mL EtOH was added 1N NaOH (6.5 mL) at 0 °C. The reaction was stirred at room temperature for 16 hrs and concentrated in vacuo. The reaction was acidified with 1N HCl to pH 2 and extracted with EtOAc. Then the EtOAc was washed with a saturated solution of $KHSO_4$, brine, dried over $Na_2SO_4$ and concentrated to give the title compound (512 mg, quantitative).
$(M+H)^+$ @ 301

D. (4-Aminobutyl)carbamic acid, phenylmethyl ester

To a solution of 1,4-diaminopentane (25 g, 155 mmol) in 100 mL DMF/$H_2O$ (1:1) was added 5N NaOH to adjust the pH to 9. CBZ-Cl (3.7 mL, 26 mmol) was added as one portion and stirred at room temperature for 16 hrs. The reaction was acidified to pH 1.25 and washed with diethyl ether. The pH of the reaction was then adjusted to 9.5 and the reaction extracted with EtOAc. The organic layer was washed with $H_2O$, brine, dried over $Na_2SO_4$ and evaporated to give title compound (1.57 g, 30%).

E. (2S-trans)-l-[[[(l,l-Dimethylethoxy)carbonyl]amino]acetyl]-4-methyl-N-[4-[[(phenylmethoxy)carbonyl]amino]-butyl]-2-piperidinecarboxamide

To a solution of Part C compound (516 mg, 1.72 mmol) and HOBT (256 mg, 1.89 mmol) in 10mL DMF was added WSC (363 mg, 1.89 mmol) and Part D CBZ amine, (381 mg, 1.72 mmol), followed by NMM (198µl, 1.89 mmol) to adjust the pH of the solution to 8. The reaction was stirred at room temperature for 16 hrs. The reaction was worked up by pouring into a saturated solution of $NaHCO_3$ and extracting with EtOAc. The EtOAc layer was washed with a saturated solution of $KHSO_4$, brine, dried over $Na_2SO_4$ and concentrated in vacuo to give the title compound (900 mg, quantitative).

F. (2S-trans)-l-(Aminoacetyl)-4-methyl-N-[4-[[(phenylmethoxy)carbonyl]amino]butyl]-2-piperidinecarboxamide, hydrochloride

To a solution of Part E compound (0.76 g, 1.51 mmol) in 5 mL MeOH was added 4.5 mL of 4N HCl/dioxane at 0 °C. After 2 hrs at room temperature, the reaction was worked up by adding $Et_2O$ to precipitate the title compound (0.70 g, quantitative).

G. (2S-trans)-4-Methyl-l-[[[(3-methyl-8-quinolinyl)sulfonyl]amino]acetyl]-N-[4-[[(phenylmethoxy)carbonyl]-amino]butyl]-2-piperidinecarboxamide

To a solution of Part F compound (500 mg, 1.13 mmol) in 11 mL $CHCl_3$ was added $Et_3N$ (475ml, 3.39 mmol) at 0 °C. After 5 minutes, 6-methyl-8-quinolinesulfonyl chloride, (273 mg, 1.13 mmol) was added. The

reaction was stirred at 0 °C for another 5 minutes then stirred at room temperature for 3 hrs. The reaction was washed with a saturated solution of $NaHCO_3$, a saturated solution of $KHSO_4$, brine, dried over $Na_2SO_4$ and evaporated to give the crude product which was purified by a silica gel column, using $CH_2Cl_2$ : MeOH eluting solvent to give the title compound (150 mg, 22%).

H. (2S-trans)-N-(4-Aminobutyl)4-methyl-l-[[[(1,2,3,4-tetrahydro-3-methyl-8-quinolinyl)sulfonyl]amino]acetyl]-2-piperidinecarboxamide, hydrochloride

A solution of Part G compound (140 mg, 0.23 mmol) and 230 µl 1N HCl in 5 mL EtOH was hydrogenated at 1 atm over 10% Pd / C (40 mg). After 16 hrs, the suspension was filtered through Celite and concentrated in vacuo to give the title free amine, (130 mg, quantitative).

I. (2S-trans)N-[4-[(Aminoiminomethyl)amino]-butyl]-4-methyl-l-[[[(l,2,3,4-tetrahydro-3-methyl-8-quinolinyl)-sulfonyl]amino]acetyl]-2-piperidinecarboxamide

To a solution of Part H compound (80 mg, 0.145 mmol) in 0.8 mL EtOH was added $Et_3N$ ( 60 ml, 0.43 mmol), followed by amidinesulfonic acid (27 mg, 0.22 mmol). After 16 hrs at room temperature, the reaction was concentrated in vacuo and was subjected to preparative HPLC using an 80:20 to 50:50 gradient ($H_2O$: $CH_3CN$) over 50 minutes to give the title compound (40 mg, 50%).
(M + H) + @ 522

| Anal Calc'd for $C_{24}H_{39}N_7O_4S$ • 1.2TFA • 1.1 $H_2O$ | | | | |
|---|---|---|---|---|
| | C, 43.54; | H, 6.04; | N, 13.46; | F, 9.39 |
| Found | C, 43.87; | H, 5.72; | N, 13.09; | F, 9.33. |

$[\alpha]_D$ = -6.7 (c = 0.51, methanol)

Example 6

trans-N-[4-[(Aminoiminomethyl)amino]butyl]-4-(methylthio)-l-[[(2-naphthalenylsulfonyl)amino]acetyl]-L-prolinamide, trifluoroacetate salt

A. cis-4-Hydroxy-l-[(phenylmethoxy)carbonyl]-L-proline, methyl ester

N-CBZ-L-keto proline was dissolved in methanol ( 150 mL), cooled to 0 to 5 °C and treated dropwise with a solution of $NaBH_4$ (2.875 g) in water (10 mL). The mixture was kept at 0 to 5 °C for 19 h. The methanol was removed in vacuo and the residue was treated with 75 mL of 3N NaOH and stirred at room temperature for 40 min. After cooling in an ice bath, the mixture was acidified with conc HCl. The product was extracted into ethyl acetate (3x50 mL). The combined extracts were washed with brine (2x40 mL), dried ($MgSO_4$ ) and freed of solvent in vacuo to give the *cis*-hydroxy acid. This was converted to the methyl ester by dissolving in methanol (75 mL), treating with acetyl chloride (7.5 mL), and stirring overnight at room temperature. The solvent was removed. The residue was dissolved in ethyl acetate (75 mL), washed with sodium bicarbonate solution (2x25mL), dried ($MgSO_4$ ) and freed of solvent in vacuo to give title compound as a viscous oil (4.315 g, 81%).

B. cis-4-[[(4-Methylphenyl)sulfonyl]oxy]-l-[(phenylmethoxy)carbonyl]-L-proline, methyl ester

The Part A alcohol (4.315g, 16.84 mmol) was dissolved in dry pyridine (23mL), cooled in an ice bath and treated with tosyl chloride (6.8g). The mixture was allowed to warm slowly to room temperature and left stirring 60 h. After cooling, cold 2N HCl was added and the mixture was left at 0 to 5 °C for 4 h. The solid was collected by filtration and dissolved in dichloromethane. This solution was washed with 1N HCl soln and brine, dried ($MgSO_4$ ) and freed of solvent in vacuo leaving title compound as a crystalline material.

C. 4-(Methylthio)-l-[(phenylmethoxy)carbonyl]-L-proline, methyl ester

Part B tosylate (2.09g, 4.8mmol) was dissolved in absolute ethanol (20mL) and acetone (20mL), under an atmosphere of argon, and treated with sodium thiomethoxide (1.39g, 19.9mmol). The mixture was heated

under reflux 1h, cooled and most of the solvent was removed in vacuo. The residue was partitioned between 1N HCl and ethyl acetate. The ethyl acetate layer was washed with water, dried ($MgSO_4$ ), and freed of solvent in vacuo. NMR and TLC indicated the material was mainly the free acid. It was converted to the methyl ester by dissolving in methanol (20 mL), adding acetyl chloride (2mL) and stirring at room temperature for two hours. After removal of the solvent, the product was purified by chromatography on silica gel, eluting with ethyl acetate:hexane (1:2) to give the title thiomethyl compound (1.364g, 99%).

D. 4-(Methylthio)-L-proline, methyl ester, hydrobromide

Part C compound (945 mg, 3.3mmol) was treated with 30% HBr in HOAc (8mL) for 1 h and then concentrated in vacuo. Trituration of the residue with ether gave a solid which was harvested by filtration and washed with more ether leaving the title deprotected amine (749 mg, 89%).

E. trans-l-[[[(l,l-Dimethylethoxy)carbonyl]-amino]acetyl]-4-(methylthio)-L-proline, methyl ester

and

F. cis-l-[N-[(l,l-Dimethylethoxy)carbonyl)glycyl]-4-(methylthio)-L-proline, methyl ester

Part D L-proline derivative (1.012 g, 3.95 mmol) and BOC-glycine (900mg, 5.1 mmol) were dissolved in DMF (20 mL) at RT. HOBT (688mg, 5.1 mmol), WSC (974mg, 5.1mmol) and NMM (1.0 mL, 9.1 mmol) were added. The reaction was stirred for 16 h, partitioned between ethyl acetate(50 mL) and 10% $KHSO_4$ (100 mL). The aqueous layer was extracted with ethyl acetate (2x50mL), and the organic layers were combined. The combined organic layers were washed with saturated $NaHCO_3$ , saturated NaCl, dried over magnesium sulfate and concentrated in vacuo to provide an oil. TLC indicated this was two major products. The crude material was purified by chromatography on silica gel. Elution with ethyl acetate:hexanes(1:2 followed by 1:1) gave the major product (tentatively assigned the *trans* configuration of the title compound, 603mg, 46%) and the minor product (tentatively assigned the *cis* configuration of the title compound, 204mg, 16%) as well as some mixed fractions (438mg, 33%).

G. trans-4-(Methylthio)-l-[[(2-naphthalenylsulfonyl)aminolacetyl]-L-proline, methyl ester

The Part E BOC derivative (600 mg, 1.8 mmol) was dissolved in trifluoroacetic acid (TFA) (15 mL) and stirred at RT 1 h. The TFA was removed by distillation under reduced pressure and by coevaporation with toluene. The crude TFA salt was dissolved in dichloromethane (20 mL), cooled in an ice bath, and 2-napthylsulfonyl chloride (Aldrich, 450mg, 2.0 mmol) was added, followed by triethylamine (1.5 mL). The solution was warmed to RT and stirred 1.5 h before diluting with dichloromethane (75 mL). This solution was washed with potassium hydrogen sulfate solution(2x40mL) and saturated $NaHCO_3$ (2 x40mL), dried over magnesium sulfate, and evaporated to provide crude title compound. The crude product was chromatographed on silica gel and eluted with 50% EtOAc in hexanes to provide title sulfonamide as a foam (700mg, 92% overall in two steps).

H. trans-4-(Methylthio)-l-[[(2-naphthalenylsulfonyl)aminolacetyl]-L-proline

A solution of Part G methyl ester (700mg, 1.65 mmol) in methanol (20 mL) was treated with 1 N NaOH solution (8mL) and stirred at room temperature for three h. After acidification with 1N HCl solution, the product was extracted into dichloromethane (2x50 mL), dried over magnesium sulfate and freed of solvent in vacuo to give title compound as a white foam (687mg, 100%).

I. trans-4-(Methylthio)-l-[[(2-naphthalenylsulfonyl)amino]acetyl]-N-[4-[[(phenylmethoxy)carbonyl]amino]butyl]-L-prolinamide

To a solution of mono-CBZ butyl diamine (484 mg, 2.18 mmol) and Part H acid (687 mg, 1.65 mmol) in DMF (25 mL) was added HOBT (230 mg, 1.7 mmol), WSC (325 mg, 1.7 mmol), and NMM (373 $\mu$L, 3.4 mmol). The reaction was stirred at RT for 20 h, partitioned between ethyl acetate (50 mL) and $KHSO_4$ solution (50 mL), back-extracted with ethyl acetate (2x50 mL), and the combined organic layers were washed with saturated $NaHCO_3$ and brine, dried ($MgSO_4$), filtered and concentrated. The crude product was chromatographed on silica gel and eluting with 50-67% EtOAc in hexanes followed by EtOAc to provide title

compound (577mg, 58%).

J. trans-N-(4-Aminobutyl)-4-(methylthio)-l-[[(2-naphthalenylsulfonyl)amino]acetyl]-L-prolinamide, hydrobromide

The CBZ group was removed from Part I compound (577 mg, 0.96mmol) by treating with 30%HBr in HOAc (10mL) for 1.5 h and then concentrated in vacuo. Trituration of the residue with ether gave a solid which was harvested by filtration and washed with more ether leaving the title deprotected amine (100%).

K. trans-N-[4-[(Aminoiminomethyl)amino]butyl]-4-(methylthio)-l-[[(2-naphthalenylsulfonyl)amino]acetyl]-L-prolinamide, trifluoroacette salt

The Part J compound (0.96 mmol) was dissolved in ethanol (20 mL), to which amidinesulfonic acid (173 mg, 1.4 mmol) and triethyl amine (375 µL, 2.7 mmol) were added. After 4 h the mixture was filtered through a pad of Celite, washed with ethanol and the filtrate was concentrated to dryness. The crude material was purified by preparative HPLC (YMC S-10 ODS 50 x 500 mm column, eluting with 54% methanol in water, containing 0.1% TFA). Fractions containing pure title compuond were combined and lyophilized to provide a white solid (279 mg, 44%), Purity ≧ 98%.
$[\alpha]_D$ = 12.7°,(c = 0.7, MeOH).

| Analysis calcd for 1.10 TFA + 0.90 $H_2O$: | | | | | |
|---|---|---|---|---|---|
| | C, 45.70; | H, 5.31; | N, 12.69; | F, 9.68; | S, 9.47. |
| Found: | C, 45.65; | H, 5.09; | N, 12.51; | F, 9.74; | S, 9.37. |

Example 7

N-[4-[(Aminoiminomethyl)amino]butyl]-l-[N-(2-naphthalenylsulfonyl)-D-phenylalanyl]-L-prolinamide, trifluoroacetate salt, hemihydrate

A. N-[4-[[(l,l-Dimethylethoxy)carbonyl]amino]butyl]-L-prolinamide, hyrochloride

A(l). (4-Aminobutyl)carbamic acid, l,l-dimethylethyl ester

To a stirred solution of 1,4-diaminobutane (50 g, 567 mmol) in 195 mL of dioxane under argon at room temperature was added dropwise a solution of Di-t-butyl dicarbonate (15.7 g, 71.9 mmol) in 195 mL of dioxane over 3.5 h. Some white precipitate appeared during the addition. The mixture was stirred at room temperature for 22 h and concentrated in vacuo. The residue was diluted with 320 mL of water and the precipitate was filtered off. The aqueous filtrate was extracted with methylene chloride (3x300 mL). The combined methylene chloride extracts were washed with water (2x200 mL) and brine (1x200 mL). The organic layer was dried ($MgSO_4$), filtered and concentrated in vacuo to give 9.79 g (72%) of title mono-BOC•amine.
TLC: silica gel, 2%$NH_4OH$ in 10%$CH_3OH/CH_2Cl_2$, $R_f$ 0.30, Ninhydrin.

A(2). N-[4-[[(l,l-Dimethylethoxy)carbonyl]amino]butyl]-l-[(phenylmethoxy)carbonyl]-L-prolinamide

To a stirred solution of N-CBZ-L-proline (12.7 g, 50.9 mmol), 1-hydroxybenzotriazole monohydrate (6.49 g, 50.9 mmol) and Part A(l) BOC amine (9.57 g, 50.9 mmol) in 250 mL of DMF was added in order 4-methylmorpholine (11.2 mL, 102 mmol) and ethyl-3-(3-dimethylamino)propyl carbodiimide hydrochloride (9.76 g, 50.9 mmol). The reaction solution was stirred at room temperature for 22 h and concentrated under pump vacuum at 50°C. The residue was diluted with 600 mL of EtOAc and washed with 1N HCl solution (2x250 mL), saturated $NaHCO_3$ solution (2x250 mL) and brine (1x250 mL). The EtOAc layer was dried ($MgSO_4$), filtered and concentrated in vacuo to give 20.7 g (97%) of title CBZ amine.
TLC: silica gel, 4%$CH_3OH/CH_2Cl_2$, $R_f$ 0.44, UV, $Ce(SO_4)$2.

A(3). N-[4-[[(l,l-Dimethylethoxy)carbonyl]amino]butyl]-L-prolinamide, hydrochloride

To a stirred solution of Part A(2) CBZ amine (20.2 g, 48.2 mmol) in 250 mL of methanol under argon was added 20%Pd(OH)2/C (4.04 g, 20% based on the weight of Part A(2) compound). The atmosphere was replaced by hydrogen with several vacuum-fill cycles. The reaction mixture was stirred at room temperature for 21 h. The catalyst was filtered off through a 4 µM polycarbonate film and rinsed with methanol (3x50 mL). The filtrate was concentrated in vacuo. The oily residue was dissolved in 200 mL of ether and treated with 1N HCl solution in ether (53.0 mL, 53.0 mmol). The solution was concentrated in vacuo. The residue was mixed with 200 mL of toluene and 30 mL of methanol and concentrated in vacuo to give title amine hydrochloride in quantitative yield (15.5 g) as an oil.

B. N-[4-[[(l,l-Dimethylethoxy)carbonyl]amino]butyl]-l-[N-[(phenylmethoxy)carbonyl]-D-phenylalanyl]-L-prolinamide

A stirred solution of N-α-CBZ-D-phenylalanine (0.56 g, 1.9 mmol) in 6.5 mL of DMF at room temperature under argon was treated with 1-hydroxybenzotriazole (0.29 g, 1.9 mmol) and ethyl-3-(3-dimethylamino)propyl carbodiimide hydrochloride (0.36 g, 1.9 mmol). After 20 minutes, Part A compound was added (0.50 g, 1.6 mmol) and stirring was carried out for 16 hours. The reaction was quenched by the addition of 75 mL of 0.25M $KHSO_4$ solution. The suspension was washed with EtOAc (2 x 40 mL), the combined EtOAc layers were washed with 0.25M $KHSO_4$ solution (2 x 40 mL), saturated aqueous $KHCO_3$ solution (2 x 40 mL), brine, dried ($Na_2SO_4$), and concentrated to yield 1.07 g of a white taffy, which by TLC analysis appeared to contain unreacted N-α-CBZ-D-phenylalanine. The crude product was redissolved in 60 mL of EtOAc, washed with saturated aqueous $KHCO_3$ solution (3 x 40 mL), brine, dried ($Na_2SO_4$), concentrated, co-evaporated several times with ether and hexane and triturated with 50 mL of hexane to yield of title compound (0.78 g, 88%) as a white solid.

C. N-[4-[[(l,l-Dimethylethoxy)carbonyl]amino]butyl]-l-D-phenylalanyl-L-prolinamide

A solution of Part B compound (0.58 g, 1.0 mmol) with 0.12 g of $Pd(OH)_2$/carbon in 5.0 mL of methanol was hydrogenated at 1 atm for 3.75 hours. The catalyst was removed by filtration through a porous membrane (Whatman 0.2µ Nylon Autovial), and the filtrate was concentrated to yield title compound (0.39 g, 88%) as a white, hygroscopic solid.

D. N-[4-[[(l,l-Dimethylethoxy)carbonyl]amino]butyl]-l-[N-(2-naphthalenylsulfonyl)-D-phenylalanyl]-L-prolinamide

Triethylamine (0.25 mL, 1.8 mmol) was added dropwise to a stirred 0°C solution of Part C compound (0.39 g, 0.90 mmol) and 2-naphthalenesulfonyl chloride (0.21 g, 0.95 mmol) in 7.0 mL of $CH_2Cl_2$. The reaction was carried out at room temperature for 45 minutes. Dichloromethane was removed under vacuum, the residue was partitioned between EtOAc (50 mL) and aqueous 0.25M $KHSO_4$ solution (15 mL), the EtOAc layer was washed with 0.25M $KHSO_4$ solution (3 x 15 mL), brine, dried ($Na_2SO_4$), and concentrated to yield 0.61 g of a colorless glass. Trituration with 50 mL of hexane yielded title compound (0.52 g, 93%) as a white solid.

E. N-(4-Aminobutyl)-1-[N-(2-naphthalenylsulfonyl)-D-phenylalanyl]-L-prolinamide, trifluoroacetate salt

Trifluoroacetic acid (5.0 mL) was added to ice-cooled Part D compound (0.48 g, 0.77 mmol). The reaction solution was stirred at room temperature for 45 minutes. Trifluoroacetic acid was removed under vacuum and co-evaporated several times with ether and hexane until title compound was obtained as a white solid (0.51 g, ca. 100%).

F. N-[4-[(Aminoiminomethyl)amino]butyl]-l-[N-(2-naphthalenylsulfonyl)-D-phenylalanyl]-L-prolinamide, trifluoroacetate salt, hemihydrate

A solution of Part E compound (0.49 g, 0.77 mmol) in 6.0 mL of absolute ethanol was treated with amidine sulfonic acid (0.14 g, 1.2 mmol) followed by triethylamine (3.2 mL, 2.3 mmol). Within several minutes reaction time, a precipitate appeared. After 5 hours reaction time, TLC analysis of the reaction mixture (silica, 3:1:1 nBuOH:HOAc:$H_2O$) indicated a small amount of unconsumed Part E compound. An

additional portion of amidine sulfonic acid (0.05 g) and triethylamine (0.53 mL) were introduced, and the reaction was continued for 16 hours. TLC analysis as above indicated complete consumption of Part E compound. The reaction mixture was concentrated, dissolved in 25 mL of $CH_3OH$, and filtered through a porous membrane (Gelman Acrodisc CR PFTE, 0.45 $\mu$). Preparative HPLC of the filtered solution (25%B to 100%B, 30 minute gradient) and subsequent lyophilization of the pooled fractions yielded title compound, (258 mg,48%) as a colorless solid. mp 100-120 °C with foaming.
$[\alpha]_D$ = +19.4° (c = 0.5, $CH_3OH$).
TLC: Rf = 0.70 (silica, 3:1:1 nBuOH ; HOAc;$H_2O$).

| Anal. Calc'd for $C_{31}H_{37}N_6O_6F_3S \bullet$ I.I $C_2HF_3O_2 \bullet 0.50\ H_2O$: | | | | | |
|---|---|---|---|---|---|
| | C, 53.60; | H, 5.49; | N, 12.02; | F, 8.97; | S, 4.59 |
| Found: | C, 53.61; | H, 5.50; | N, 11.81; | F, 9.03; | S, 4.36 |

Example 8

N-[4-[(Aminoiminomethyl)amino]butyl]-l-[N-(2-naphthalenylsulfonyl)-D-glutaminyl]-L-prolinamide

A. N-[4-[[(l,l-Dimethylethoxy)carbonyl]amino]butyl]-l-[N-[(phenylmethoxy)carbonyl]-D-glutaminyl]-L-prolinamide

To a stirred solution of Example 7 Part A amine hydrochloride (0.56 g, 1.74 mmol), 1-hydroxybenzotriazole monohydrate (0.29 g, 1.74 mmol) and N-CBZ-D-glutamine (0.49 g, 1.74 mmol) in 15 mL of DMF was added 4-methylmorpholine (0.57 mL, 5.23 mmol) followed by ethyl-3-(3-dimethylamino)propyl carbodiimide hydrochloride (0.33 g, 1.74 mmol). The solution was stirred at room temperature for 19 h and concentrated under pump vacuum at 45 °C. The oily residue was diluted with 160 mL of EtOAc and washed with 1N HCl solution (2x60 mL), saturated $NaHCO_3$ solution (2x60 mL) and brine (1x60 mL). The EtOAc layer was dried ($MgSO_4$), filtered and concentrated in vacuo to give 0.75 g (77%) of title CBZ amine.
TLC: silica gel, 6%$CH_3OH/CH_2Cl_2$, $R_f$ 0.22, $Ce(SO_4)_2$.

B. N-[4-[[(l,l-Dimethylethoxy)carbonyl]amino]butyl]-l-[N-(2-naphthalenylsulfonyl)-D-glutaminyl]-L-prolinamide

To a stirred solution of Part A CBZ amine (0.73 g, 1.33 mmol) in 12 mL of methanol under argon was added $Pd(OH)_2/C$ (146 mg, 20% based on the weight of Part A compound). The atmosphere was replaced by hydrogen with several vacuum-fill cycles. The reaction solution was stirred at room temperature for 22 h and the catalyst was filtered off through a 4$\mu$M polycarbonate film. The solid was rinsed with methanol (2x20 mL). The filtrate was concentrated in vacuo to give 0.54 g of the intermediate amine. To a stirred solution of this amine (0.44 g, 1.07 mmol) and 2-naphthalenesulfonyl chloride (0.27 g , 1.18 mmol, in 10 mL of dry methylene chloride under argon at 0 °C was added $Et_3N$ (0.33 mL, 2.34 mmol). The reaction solution was stirred at room temperature for 2 h and diluted with 180 mL of EtOAc. This solution was washed with 1N HCl solution (2x60 mL), saturated $NaHCO_3$ solution (1x60 mL) and brine (1x60 mL). The organic layer was dried ($MgSO_4$), filtered and concentrated in vacuo. Purification was effected by flash chromatography on 30 g of Merck silica gel 60 using 240 mL of 4%$CH_3OH/CH_2Cl_2$ and 480 mL of 6%$CH_3OH/CH_2Cl_2$ as eluants to give pure title sulfonamide (310 mg, 48%).
TLC: silica gel, 4%$CH_3OH/CH_2Cl_2$, $R_f$ 0.20, UV, $I_2$.

C. N-[4-[(Aminoiminomethyl)amino]butyl]-l-[N-(2-naphthalenylsulfonyl)-D-glutaminyl]-L-prolinamide

To a stirred solution of Part B sulfonamide (310 mg, 0.51 mmol) in 5 mL of dry methylene chloride was added 0 °C 4N HCl in dioxane (6.00 mL, 24.0 mmol). The solution was stirred at room temperature for 2 h and diluted with 120 mL of ether. The solid was filtered off and rinsed with ether (2x30 mL). The solid was dissolved in 60 mL of methanol and concentrated in vacuo to give the intermediate amine hydrochloride. To a stirred mixture of this amine hydrochloride and $Et_3N$ (0.39 mL, 2.31 mmol) in 5 mL of absolute EtOH under argon was added aminoiminomethanesulfonic acid (204 mg, 1.64 mmol). The mixture was stirred at room temperature for 5 h and concentrated in vacuo. The reaction mixture was purified by preparative HPLC, eluting isocratically with a 37% composition of water:methanol (90:10 and 10:90) containing 0.2% $H_3PO_4$. The fractions were concentrated in vacuo and lyophilized to give 260 mg (93%) of title compound.

m.p. 72-74 °C
$[\alpha]_D$ = +5.80 (c = 0.69, methanol).

| Anal. Calc'd for $C_{25}H_{35}N_7O_5S \cdot 1.60\ TFA \cdot 1.0\ H_2O$: | | | | | |
|---|---|---|---|---|---|
| | C, 45.40; | H, 5.21; | N, 13.14; | S, 4.30; | F, 12.22 |
| Found: | C, 45.45; | H, 4.98; | N, 12.95; | S, 4.32; | F, 11.90 |

Following the procedures of Examples 1 to 8 and those outlined above, the following additional examples may be prepared.

| | Melting Point °C, Optical Rotation and/or Elemental Analysis (where available) |
|---|---|
| 9. [2R-[1(S*),2α,4β)]-N-[3-[(Aminoiminomethyl)amino]propyl]-1-[4-hydroxy-3-[(2-naphthalenylsulfonyl)amino)-1,2-dioxobutyl]-2-piperidinecarboxamide, trifluoroacetate (1:1) salt | $[\alpha]_D$=+11.0 (c=1.09 MeOH) |

Anal. Calc'd for $C_{24}H_{34}N_6O_5S \cdot 1.1TFA \cdot 1.34\ H_2O$:
C, 47.10; H, 5.70; N, 12.58; S, 4.80; F, 9.38
Found: C, 47.39; H, 5.41; N, 12.29; S, 5.21; F, 9.56

| | |
|---|---|
| 10. N-[4-[(Aminoiminomethyl)-amino]butyl]-1-[N-(2-naphthalenyl-sulfonyl)-L-seryl]-L-prolinamide | $[\alpha]_D$=-70.2 (c=0.5, MeOH) |

Anal. Calc'd for $C_{23}H_{32}N_6O_5S \cdot 1.15\ TFA \cdot 0.5\ H_2O$:
C, 47.13; H, 5.34; N, 13.03; S, 4.97; F, 10.17
Found: C, 46.83; H, 5.28; N, 12.84; S, 5.14; F, 10.43

| | |
|---|---|
| 11. N-[4-[(Aminoiminomethyl)-amino]butyl]-1-[N-(2-naphthalenyl-sulfonyl)-L-seryl]-D-prolin-amide, trifluoroacetate (1:1) salt | $[\alpha]_D$=-14.8 (c=0.5, MeOH) |

Anal. Calc'd for $C_{23}H_{32}N_6O_5S \cdot 1.15\ TFA \cdot 1.6\ H_2O$:
C, 45.73; H, 5.51; N, 12.65; S, 4.82; F, 9.86
Found: C, 45.58; N, 5.19; N, 12.41; S, 5.14; F, 10.01

12. (2R-trans)-N-[4-[(Aminoiminomethyl)amino]butyl]-4-methyl-1-[[(2-naphthalenylsulfonyl)amino]-acetyl]-2-piperidinecarboxamide, trifluoroacetate salt, hydrate — $[\alpha]_D$=+33.0 (c=1.08 MeOH)

Anal. Calc'd for $C_{24}H_{34}N_6O_4S \cdot 1.35TFA \cdot 0.85\ H_2O$:
C, 47.73; H, 5.56; N, 12.51; S, 4.77; F, 11.45
Found: C, 47.73; H, 5.51; N, 12.13; S, 4.95; F, 11.42

13. [2R-[1(S*)2α,4β]]-N-[4-[(Aminoiminomethyl)amino]butyl]-1-[3-hydroxy-2-[(2-naphthalenylsulfonyl)amino]-1-oxopropyl]-4-methyl-2-piperidinecarboxamide, trifluoroacetate salt, hydrate — $[\alpha]_D$=+18.8 (c=1.06 MeOH)

Anal. Calc'd for $C_{26}H_{38}N_6O_5S \cdot 1.36TFA \cdot 1.00\ H_2O$:
C, 47.93; H, 5.79; N, 11.68; S, 4.45; F, 10.77
Found: C, 47.92; H, 5.66; N, 11.53; S, 4.89; F, 10.75

14. [1(S)]-N-[4-[(Aminoiminomethyl)amino]butyl]-1,2,3,4-tetrahydro-2-[3-hydroxy-2-[(2-naphthalenylsulfonyl)amino]-1-oxopropyl]-3-isoquinolinecarboxamide, isomer A, trifluoroacetate salt, hydrate — $[\alpha]_D$=-30.9 (c=0.5, MeOH)

Anal. Calc'd for $C_{28}H_{34}N_6O_5S \cdot 1.25\ TFA \cdot 1.0\ H_2O$:
C, 50.37; H, 5.16; N, 11.56; S, 4.41; F, 9.80
Found: C, 50.31; H, 5.05; N, 11.58; S, 4.44; F, 9.65

15. [1(S)]-N-[4-[(Aminoiminomethyl)amino]butyl]-1,2,3,4-tetrahydro-2-[3-hydroxy-2-[(2-naphthalenylsulfonyl)amino]-1-oxopropyl]-3-isoquinolinecarboxamide, isomer B, trifluoroacetate salt, hydrate — $[\alpha]_D$=-22.8 (c=0.6, MeOH)

Anal. Calc'd for $C_{28}H_{34}N_6O_5S \cdot 1.25$ TFA$\cdot$1.0 $H_2O$:
C, 50.15; H, 5.13; N, 11.47; S, 4.37; F, 10.11
Found: C, 50.04; H, 4.95; N, 11.36; S, 4.51; F, 10.17

16. (4S*)-N-[4-[(Aminoiminomethyl)amino]butyl]-4-hydroxy-1-[N-(2-naphthalenylsulfonyl)-L-seryl]-L-prolinamide, trifluoroacetate (1:1) salt — $[\alpha]_D$=-29.0 (c=0.6, MeOH)

Anal. Calc'd for $C_{23}H_{32}N_6O_6S \cdot 1.15$ TFA$\cdot$1.0 $H_2O$:
C, 45.25; H, 5.31; N, 12.51; S, 4.77; F, 9.76
Found: C, 45.28; H, 4.98; N, 12.52; S, 4.86; F, 9.86

17. (4S*)-N-[4-[(Aminoiminomethyl)amino]butyl]-4-methoxy-1-[N-(2-naphthalenylsulfonyl)-L-seryl]-L-prolinamide, trifluoroacetate (1:1) salt — $[\alpha]_D$=-24.5 (c=0.6, MeOH)

Anal. Calc'd for $C_{24}H_{34}N_6O_6S \cdot 1.1$ TFA$\cdot$1.3 $H_2O$:
C, 46.04; H, 5.56; N, 12.30; S, 4.69; F, 9.17
Found: C, 46.04; H, 5.48; N, 12.16; S, 4.78; F, 9.22

18. N-[4-[(Aminoiminomethyl)-amino]butyl]-1-[N-(2-naphthalenylsulfonyl)-L-phenylalanyl]-L-prolinamide, trifluoroacetate (1:1) salt — m.p. 172-178°C with foaming; $[\alpha]_D$=-27.0 (c=0.5, $CH_3OH$)

Anal. Calc'd for $C_{31}H_{37}N_6O_6F_3S$•1.2 $C_2HF_3O_2$•0.80 $H_2O$:
C, 52.68; H, 5.46; N, 11.74; F, 9.55; S, 4.48
Found: C, 52.71; H, 5.35; N, 11.50; F, 9.33; S, 4.26

19. (2S-trans)-N-[4-[(Aminoiminomethyl)amino]butyl]-4-methyl-1-[[(2-naphthalenylsulfonyl)amino]-acetyl]-2-piperidinecarboxamide — $[\alpha]_D$=-36.4 (c=0.98, MeOH)
Anal. Cald'd for $C_{26}H_{35}N_6O_6S$•0.25TFA•1.20 $H_2O$:
C, 47.74; H, 5.69; N, 12.60; F, 10.68; S, 4.81
Found: C, 47.75; H, 5.51; N, 12.62; F, 10.86; S, 4.95

20. N-[4-[(Aminoiminomethyl)-amino]butyl]-1-[N-(2-naphthalenylsulfonyl)-L-glutaminyl]-L-prolinamide, trifluoroacetate (1:1) salt — $[\alpha]_D$=-48.1 (c=0.68, methanol)
Anal. Calc'd for $C_{25}H_{35}N_7O_5S$•1.27 TFA•1.57 $H_2O$:
C, 46.02; H, 5.53; N, 13.64; S, 4.46; F, 10.07
Found: C, 46.02; H, 5.18; N, 13.33; S, 4.86; F, 10.06

21. N-[4-[(Aminoiminomethyl)-amino]butyl]-1-[N-(2-naphthalenylsulfonyl)-L-threonyl]-L-prolinamide, trifluoroacetate (1:1) salt — m.p. 68-78°C, $[\alpha]_D$=-72.6 (c=0.69, methanol)
Anal. Calc'd for $C_{24}H_{34}N_6O_5S$•1.25 TFA•1.20 $H_2O$:
C, 46.62; H, 5.56; N, 12.31; S, 4.70; F, 10.43
Found: C, 46.75; H, 5.50; N, 12.08; S, 4.82; F, 10.40

22. N-[4-[(Aminoiminomethyl)-amino]butyl]-1-[N-(2-naphthalenylsulfonyl)-L-allothreonyl]-L-prolinamide, trifluoroacetate (1:1) salt — m.p. 85-96°C, $[\alpha]_D$=-70.8 (c=0.76, methanol)

Anal. Calc'd for $C_{24}H_{34}N_6O_5S$•1.16 TFA•0.97 $H_2O$:
C, 47.30; H, 5.59; N, 12.57; S, 4.80; F, 9.89
Found: C, 47.30; H, 5.16; N, 12.39; S, 5.19; F, 9.48

23. (S)-N-[4-[(Aminoiminomethyl)-amino]butyl]-4-(methylthio)-1-[N-(2-naphthalenylsulfonyl)glycyl]-L-prolinamide, trifluoroacetate (1:1) salt — $[\alpha]_D$=+13.4 (c=0.6, MeOH)

Anal. Calc'd for $C_{23}H_{32}N_6O_5S_2$•1.10 TFA•1.0 $H_2O$:
C, 45.58; H, 5.33; N, 12.65; S, 9.44; F, 9.66
Found: C, 45.52; H, 5.11; N, 12.46; S, 9.49; F, 9.65

24. (R)-N-[4-[(Aminoiminomethyl)-amino]butyl]-4-(methylthio)-1-[N-(2-naphthalenylsulfonyl)glycyl]-L-prolinamide, trifluoroacetate (1:1) salt — $[\alpha]_D$=-12.7 (c=0.7, MeOH)

Anal. Calc'd for $C_{23}H_{32}N_6O_4S_2$•1.10 TFA•0.9 $H_2O$:
C, 45.70; H, 5.31; N, 12.69; S, 9.47; F, 9.68
Found: C, 45.65; H, 5.09; N, 12.51; S, 9.37; F, 9.74

25. N-[4-[(Aminoiminomethyl)-amino]butyl]-1-[[(2-naphthalenyl-sulfonyl)amino]acetyl]-2-piperidinecarboxamide

High Resolution Mass Spectrum: $(M+H)^+$=489.2287.

26. N-[4-[(Aminoiminomethyl)-amino]butyl]-1-[N-(2-naphthalenylsulfonyl)-D-tryptophyl]-L-prolinamide, trifluoroacetate (1:1) salt — m.p.100-160°C foam/dec; $[\alpha]_D$=+44.4 (c=0.5, $CH_3OH$)

Anal. Calc'd for $C_{31}H_{37}N_7O_4S \cdot 1.06\ C_2HF_3O_2 \cdot 1.35\ H_2O$:
C, 53.12; H, 5.49; N, 13.09; F, 8.07; S, 4.28
Found: C, 53.51; H, 5.35; N, 12.73; F, 8.10; S, 4.28

27. (2S-trans)-N-[4-[(Aminoiminomethyl)amino]butyl]-4-methyl-1-[[[(3-methyl-8-quinolinyl)-sulfonyl]amino]acetyl]-2-piperidinecarboxamide, trifluoroacetate salt — $[\alpha]_D$=-24.1 (c=0.99 MeOH)

Anal. Calc'd for $C_{24}H_{35}N_7O_4S \cdot 1.37TFA \cdot 1.00\ H_2O$:
C, 46.42; H, 5.59; N, 14.17; F, 11.29; S, 4.63
Found: C, 46.51; H, 5.32; N, 14.06; F, 11.28; S, 4.55

28. N-[4-[(Aminoiminomethyl)-amino]butyl]-1-[[(8-quinolinyl-sulfonyl)amino]acetyl]-2-piperidinecarboxamide, trifluoroacetate salt

Anal. Calc'd for $C_{22}H_{31}N_7O_6S \cdot 2C_2HF_3O_2 \cdot 2.20\ H_2O$:
C, 41.21; H, 5.04; N, 12.84; F, 14.93; S, 4.20
Found: C, 41.34; H, 4.69; N, 12.39; F, 14.91; S, 4.55

29. N-[4-[(Aminoiminomethyl)-amino]butyl]-1-[N-(2-naphthalenylsulfonyl)-D-α-glutamyl]-L-prolinamide, trifluoroacetate (1:1) salt — $[\alpha]_D$= (c=0.60, methanol)

Anal. Calc'd for $C_{25}H_{34}N_6O_6S \cdot 1.35\ TFA \cdot 1.13\ H_2O$:
C, 46.42; H, 5.31; N, 12.16; S, 4.35; F, 10.43
Found: C, 46.42; H, 5.33; N, 11.84; S, 4.66; F, 10.50

Example 29

N-[4-[(Aminoiminomethyl)amino]butyl]-l-[N-(methylsulfonyl)-D-phenylalanyl]-L-prolinamide, trifluoroacetate (l:l) salt

A. N-[4-[(Aminoiminomethyl)amino]butyl]-l-D-phenylalanyl-L-prolinamide, trifluoroacetate salt

A(l). N-[4-(Aminobutyl)]-l-[N-[(phenylmethoxy)carbonyl]-D-phenylalanyl-L-prolinamide, trifluoroacetate

The title compound of Example 7, Part B (0.78 g, 1.38 mmol) was dissolved in trifluoroacetic acid (3.2 mL)). After 3 hours the solvent was evaporated and then co-evaporated with hexane/ether five times, and dried 16 hours in vacuo to provide a colorless taffy which was used in the next step.

A(2). N-[4-[(Aminoiminomethyl)amino]butyl]-l-[N-[(phenylmethoxy)carbonyl]-D-phenylalanylL-prolinamide, trifluoroacetate

The Part A(l) compound (0.80 g, l.38 mmol) was dissolved in absolute ethanol (10.9 mL) and treated with amidinesulfonic acid (0.26 g, 1.5 eq), followed by triethylamine (0.58 mL, 3 eq). After stirring for two hours the solvent was evaporated and the crude product purified on reverse phase HPLC to provide a colorless solid (4l4 mg, 45%).

A(3). N-[4-[(Aminoiminomethyl)amino]butyl]-l-D-phenylalanyl-L-prolinamide, trifluoroacetate salt

The title compound of Part A(2) (0.20 g, 0.30 mmol) was dissolved in methanol (1.5 mL) and hydrogenated at l atmosphere and room temperature for 3 hours with $Pd(OH)_2$ (40 mg of 20% catalyst). The catalyst was removed by filtration, and the solvent stripped to provide an oil. The oil was dissolved in methanol, acidified with trifluoroacetic acid (0.2 mL) and evaporated to dryness, then dissolved in water and lyophilized to provide the title compound (152 mg).
Optical rotation: = -75.8° (c=0.5, $CH_3OH$).

| Anal. Calc'd for $C_{19}H_{30}N_6O_2 \cdot 2.07\ C_2HF_3O_2 \cdot 0.90\ H_2O$): | | | | |
|---|---|---|---|---|
| | C, 44.35; | H, 5.45; | N, l3.4l; | F, l8.82 |
| Found: | C, 44.52; | H, 5.0l; | N, l3.26; | F, l8.52. |

B. N-[4-[(Aminoiminomethyl)amino]butyl]-l-[N-(methylsulfonyl)-D-phenylalanyl]-L-prolinamide, trifluoroacetate (l:l) salt

To a stirred solution of Part A amine (550 mg, 0.80 mmol) in 15 mL of dry $CH_2Cl_2$ and 15 mL of dry THF under argon was added in order triethylamine ($Et_3N$) (0.44 mL, 3.20 mmol) and methanesulfonyl chloride (68.0 µL, 0.88 mmol). This turbid mixture was stirred at room temperature for 3 h and diluted with 0.50 mL of water. The mixture was stirred at room temperature for 10 min and concentrated in vacuo. The residue was diluted with 30 mL of methanol, concentrated in vacuo and purified by preparative HPLC. The fractions were concentrated in vacuo and lyophilized to give 300 mg (75%) of title compound.
Opt. rot.: $[\alpha]_D$ = -68.7° (C=l.00, methanol).

| Elemental Analysis (%) Calc'd: | | | | | |
|---|---|---|---|---|---|
| | C, 43.59; | H, 5.3l; | N, l3.09; | S, 4.99; | F, l4.65 |
| Found: | C, 43.59; | H, 5.33; | N, l3.08; | S, 4.80; | F, l4.69 |

Example 30

N-[[l-(Aminoiminomethyl)-4-piperidinyl]methyl]-l-[N-(methylsulfonyl)-D-phenylalanyl]-L-prolinamide

A. 4-(Aminomethyl)-N,N'-bis[(l,l-dimethylethoxy)carbonyl]-l-piperidinecarboximidamide

To a stirred solution of 4-aminomethylpiperidine (0.72 g, 6.31 mmol) in 40 mL of toluene was added benzaldehyde (0.78 mL, 6.94 mmol). The reaction solution was refluxed for 18 h and water was removed by a Dean Stark trap. The reaction solution was cooled to room temperature at which time bis-Boc amidinopyrazole (1.96 g, 6.31 mmol) was added. The reaction solution was stirred at room temperature for 48 h and concentrated in vacuo. The oily residue was diluted with 15 mL of 1M (aq) KHSO4 solution and stirred at room temperature for 5 h. This aqueous solution was washed with ether (2x20 mL) and basified to pH 12 by the addition of 1N NaOH solution. This basic solution was then saturated with NaCl and extracted with dichloromethane (3x60 mL). The combined dichloromethane extracts were dried ($MgSO_4$), filtered and concentrated in vacuo to give 2.10 g (93%) of title amine which was used for the next transformation without further purification.

B. (S*)-N-[[l-[[[(l,l-Dimethylethoxy)carbonyl]amino][[(l,l-dimethylethoxy)carbonyl]imino]methyl]-4-piperidinyl]-methyl]-l-[l-[[(l,l-dimethylethoxy)carbonyl]aminol-2-phenylethyl]-L-prolinamide

To a stirred solution of N-Boc-D-Phe-L-Pro-OH (0.73 g, 2.02 mmol), Part A amine (0.72 g, 2.02 mmol) and 1-hydroxybenzotriazole monohydrate (0.34 g, 2.02 mmol) in 30 mL of DMF was added in order 4-methylmorpholine (0.66 mL, 6.05 mmol) and ethyl-3-(3-dimethylamino)propyl carbodiimide hydrochloride (0.39 g, 2.02 mmol). The reaction solution was stirred at room temperature for 19 h and concentrated under pump vacuum at 45 °C. The residue was diluted with 100 mL of saturated $NaHCO_3$ solution and extracted with dichloromethane (4x100 mL). The combined dichloromethane extracts were dried ($MgSO_4$), filtered and concentrated in vacuo. This was chromatographed on silica gel to give 0.70 g (50%) of title bis-Boc guanidine.

C. N-[[l-(Aminoiminomethyl)-4-piperidinyl]methyl]-l-D-phenylalanyl-L-prolinamide

To a stirred solution of Part B bis-Boc guanidine (0. 68 g, 0.97 mmol) in 6.0 mL of dichloromethane was added trifluoroacetic acid (TFA) (6.00 mL, 77.9 mmol). The reaction solution was stirred at room temperature for 3 h and concentrated in vacuo. This was purified by prep HPLC to give 310 mg (45%) of title compound.

D. N-[[l-(Aminoiminomethyl)-4-piperidinyl]methyl]-l-[N-(methylsulfonyl)-D-phenylalanyl]-L-prolinamide

To a stirred solution of Part C guanidine (275 mg, 0.68 mmol) in 4.0 mL of dichloromethane and 2.0 mL of THF under argon at 0 °C was added $Et_3N$ (0.38 mL, 2.74 mmol) and methanesulfonyl chloride (69 $\mu$L) in order. The reaction mixture was stirred at room temperature for 2.5 h and diluted with 2.0 mL of water. The mixture was concentrated in vacuo, and purified by prep HPLC to give 160 mg (37%) of title compound.

Example 31

N-[[l-(Aminoiminomethyl)-3-piperidinyl]methyl]-l-[N-(methylsulfonyl)-D-phenylalanyl]-L-prolinamide

A. N-Boc-3-hydroxymethylpiperidine

To a stirred solution of 3-hydroxymethylpiperidine (15.1 g, 131 mmol) and $Et_3N$ (21.9 mL, 158 mmol) in 100 mL of dichloromethane was added dropwise a solution of di-t-butyl dicarbonate (31.5 g, 144 mmol) in 100 mL of dichloromethane over 1 h. The reaction was stirred at room temperature for 18 h and then diluted with 200 mL of dichloromethane. The resulting solution was washed with 1N HCl solution (3x100 mL), saturated $NaHCO_3$ solution (2x100 mL) and brine (1x100 mL). The organic layer was dried ($MgSO_4$), filtered and concentrated in vacuo to give N-Boc-3-hydroxymethylpiperidine (27.0 g, 96%).

B. 3-(Azidomethyl)-l-piperidinecarboxylic acid, 1,1-dimethylethyl ester

To a stirred solution of Part A N-Boc-3-hydroxymethylpiperidine (27.0 g, 126 mmol) in 150 mL of dry dichloromethane under argon at 0°C was added in order triethylamine (22.7 mL, 163 mmol) and methanesulfonyl chloride (11.7 mL, 151 mmol). The reaction was stirred at room temperature for 1.5 h and diluted with 450 mL of dichloromethane. The reaction was washed with 0°C 1N HCl solution (2x100 mL) and brine (1x100 mL). The dichloromethane layer was dried ($Na_2SO_4$), filtered and concentrated in vacuo. The residue was dissolved in 200 mL of DMF and combined with sodium azide (24.5 g, 377 mmol). The mixture was stirred at room temperature for 33 h and the solid was filtered off. The filtrate was concentrated under pump vacuum at 45°C. The residue was partitioned between 400 mL of EtOAc and 10% sodium thiosulfate solution (2x100 mL) and brine (1x100 mL). The EtOAc layer was dried ($MgSO_4$), filtered and concentrated in vacuo. Purification was effected by a flash column chromatography on silica gel to give 19.5 g (65%) of title azide.

C. 3-(Aminomethyl)-l-piperidinecarboxylic acid, l,l-dimethylethyl ester

To a stirred solution of Part B azide (19.0g, 79.2 mmol) in 250 mL of methanol under argon was added 10%Pd/C (3.80 g, 20% based on the weight of Part B azide). The atmosphere was replaced with hydrogen by several vacuum-fill cycles. The mixture was stirred at room temperature for 15 h. The catalyst was filtered through a 4µM polycarbonate film and rinsed with methanol (4x30 mL). The filtrate was concentrated in vacuo to give 16.3 g (96%) of title amine.

D. N-[[l-[(l,l-Dimethylethoxy)carbonyl]-3-piperidinyl]methyl]-l-[N-[(phenylmethoxy)carbonyl]-D-phenylalanyl]-L-prolinamide

To a stirred solution of Part C amine (2.00 g, 9,35 mmol), N-Cbz-D-Phe-L-Pro (3.70 g, 9.35 mmol), 1-hydroxybenzotriazole monohydrate (1.58 g, 9.35 mmol) and 4-methylmorpholine (3.07 mL, 28.0 mmol) was added ethyl-3-(3-dimethylamino)propyl carbodiimide hydrochloride (1.79 g, 9.35 mmol). The reaction solution was stirred at room temperature for 17 h and concentrated under pump vacuum at 45°C. The residue was dissolved in 360 mL of EtOAc and washed with 1N HCl solution (2x120 mL), saturated $NaHCO_3$ solution (1x120 mL) and brine (1x120 mL). The EtOAc layer was dried ($MgSO_4$), filtered, concentrated in vacuo and chromatographed on silica gel to give 1.30g (23%) of title carbamate.

E. N-[[l-(Aminoiminomethyl)-3-piperidinyl]methyl]-l-[N-[(phenylmethoxy)carbonyl]-D-phenylalanyl]-L-prolinamide

To a stirred solution of Part D carbamate (2.30 g, 3.89 mmol) in 10 mL of dry dichloromethane was added 0°C 4N HCl in dioxane (15.0 mL, 60.0 mmol). The solution was stirred at room temperature for 3 h and diluted with 300 mL of ether. The precipitate was filtered off and rinsed with ether (3x30 mL). The precipitate was dried under pump vacuum at room temperature and purified by prep HPLC to give 1.39 g (59%) of intermediate amine•TFA salt. To a stirred solution of the intermediate amine•TFA salt (500 mg, 0.83 mmol) and diisopropylethyl amine (0.35 mL, 1.98 mmol) in 2.0 mL of DMF was added 1H-pyrazole-1-carboxamidine (133 mg, 0.91 mmol). The reaction solution was stirred at room temperature for 6 h and diluted with 100 mL of ether. The desired oily precipitate was separated from the ether solution and purified by prep HPLC to give 250 mg (47%) of title Cbz-carbamate.

F. N-[[l-(Aminoiminomethyl)-3-piperidinyl]methyl]-l-D-phenylalanyl-L-prolinamide

To a stirred solution of Part E Cbz-carbamate (240 mg, 0.37 mmol) in 10 mL of methanol under argon was added 20%Pd(OH)2/C (48 mg, 20% based on the weight of Part E Cbz-carbamate). The atmosphere was replaced with hydrogen by several vacuum-fill cycles. The reaction mixture was stirred at room temperature for 24 h. The catalyst was filtered off and rinsed with methanol (4x20 mL). The filtrate was concentrated in vacuo. The residue was dissolved in 50 mL of a solution of 0.1%TFA in water and lyophilized to give 220 mg (82%) of title compound.

G. N-[[l-(Aminoiminomethyl)-3-piperidinyl]methyl]-l-[N-(methylsulfonyl)-D-phenylalanyl]-L-prolinamide

Following the procedure of Example 30 Part D except substituting the Part F amidine for the Example 30 Part C amidine, the title compound is obtained.

Example 32

N-[[l-(Aminoiminomethyl)-4-piperidinyl]methyl]-l-[N-[(phenylmethyl)sulfonyl]-D-alanyl]-L-prolinamide, trifluoroacetate (l:l) salt

A. N-[[(Phenylmethoxy)-carbonyl]-D-alanyl]-L-proline methyl ester

To a solution of N-CBZ-D-alanine (28.1 g, 0.149 mol) in DMF (250 mL) at 0°C, was added L-proline-methylester.HCl (24.6 g, 0.149 mmol), 1-hydroxybenzotriazole hydrate (22.2 g, 0.164 mmol), ethyl-3-(3-dimethylamino)-propyl carbodiimide.HCl (31.4 g, 0.164 mmol) and 4-methylmorpholine ( 22.6 g, 0.224 mmol). The reaction mixture was stirred for 12 h while allowing the reaction to warm to room temperature. The reaction mixture was poured in water (750 mL) and extracted with ethyl acetate (2x150 mL). The combined organic extracts were washed as follows: $KHSO_4$ (0.25 M, 2x50 mL), water (1x50 mL), saturated aqueous $NaHCO_3$ (2x50 mL) and saturated NaCl (1x50 mL). The organic layer was dried over $MgSO_4$, filtered and the solvent removed in vacuo to give the title compound, 41.16 g (92%), as an oil. MS $(M+H)^+ = 301^+$.

B. N-[[[(Phenylmethyl)sulfonyl]carbonyl]-D-alanyl]-L-proline methyl ester

A solution of Part A compound (1.56 g, 5.2 mmol) in trifluoroacetic acid (3 mL) was stirred at 0°C for 1.5 hr and and concentrated in vacuo to give the corresponding TFA salt. This salt was dissolved in chloroform (10 mL), and triethylamine (2.2 mL, 15.6 mmol) and benzylsulfonyl chloride (1.48, 7.8 mmol) at 0°C were added to it. The reaction mixture was stirred to room temperature overnight. After 25 h, the reaction mixture was poured into EtOAc (50 mL), washed with 0.25 M aqueous $KHSO_4$ (2x20 mL), $H_2O$ (1x20 mL) and saturated aqueous $NaHCO_3$ (2x20 mL), successively; dried over $MgSO_4$, filtered and concentrated in vacuo to give title compound as an oil (1.56 g, 85%): MS $(M + H)^+ = 355^+$.

C. N-[[[(Phenylmethyl)sulfonyl]carbonyl]-D-alanyl]-L-proline

To a solution of Part B compound (1.33 g, 3.75 mmole) in THF (10 mL) at 0°C was added aqueous NaOH (1.0 N, 9.5 mL). After 30 min the reaction mixture was warmed to room temperature and stirring continued for an additional 4 h. The organic solvent was removed in vacuo. The aqueous layer was acidified to pH of 2-3 and extracted with ethyl acetate (3x10 mL). The combined extracts were dried over $MgSO_4$, filtered and the solvent removed in vacuo to give title compound, 1.21 g (94%), as a white solid.
MS $(M+H)^+ = 341^+$.

D. N-[[[1-(1,1-Dimethylethoxy)carbonyl]-4-piperidinyl]methyl]-1-[N-[(phenylmethyl)sulfonyl]-D-alanyl]-L-prolinamide

To a solution of Part C compound (1.09 g, 3.2 mmol) in DMF (15 mL) at 0°C were added N-l,l-[-(dimethylethoxy)carboxyl]-4-methylaminopiperidine (prepared in Example 33 Part A) (0.685 g, 3.2 mmol), 1-hydroxybenzotriazole hydrate (0.476 g, 3.5 mmol), ethyl-3-(3-dimethylamino)-propyl carbodiimide.HCl (0.675 g, 3.5 mmol) and 4-methylmorpholine untill basic by pH paper. The reaction mixture was stirred for 12 h while allowing the reaction to warm to room temperature. The reaction mixture was poured into 0.25 M aqueous $KHSO_4$ (50 mL) and extracted with EtOAc (2x20 mL). The organic layers were combined and washed with 0.25 M aqueous $KHSO_4$ (1x30 mL), water (1x25 mL), saturated aqueous $NaHCO_3$ (2x25 mL) and water (1x20 mL). The organic layer was dried over $MgSO_4$, filtered and the solvent removed in vacuo to give title compound, 1.67 g (9%), as an oil.
MS $(M+H)^+ = 537^+$.

E. N-[[1-(Aminoiminomethyl)-4-piperidinyl]methyl]-1-[N-[(phenylmethyl)sulfonyl]-D-alanyl]-L-prolinamide, trifluoroacetate (1:1) salt

A solution of Part D compound (1.17 g, 2. 2 mmol) in trifluoroacetic acid (10.0 mL) was stirred at 0°C for 5 h. The reaction mixture was concentrated in vacuo to give the corresponding TFA salt. To a solution of this salt (2.2 mmol), 1H-pyrazole-1-carboxamidine hydrochloride (0.474 g, 3.3 mmol) and N,N-diisopropylethylamine (1.1 mL, 6.5 mmol) in DMF (3.0 mL) were added and stirred for 3 days. The reaction mixture was concentrated in vacuo and purified by preparative HPLC ($CH_3CN/H_2O$ with TFA buffer using a C-18 silica gel column). The appropriate fractions were combined, concentrated in vacuo, dissolved in $H_2O$ and lyophilized to yield title compound (0.486 g, 38 %) as a white solid:
$[a]_D$ = +1.4 (c 1.0, MeOH)
MS $(M + H)^+ = 479^+$

| Anal. Calc'd for $C_{22}H_{34}N_6O_4S$ • 1.3 TFA • 0.7 $H_20$: | | | | | |
|---|---|---|---|---|---|
| | C, 46.21; | H, 5.78; | N, 13.14; | S, 5.01; | F, 11.59 |
| Found: | C, 46.42; | H, 5.83; | N, 12.88; | S, 4.97; | F, 11.82 |

Example 33

N-[[l-(Aminoiminomethyl)-4-piperidinyl]methyl]-l-[N-[(2,2,2-trifluoroethyl)sulfonyl]-D-phenylalanyl]-L-prolinamide, trifluoroacetate (l:l) salt

A. N-1,1-[(Dimethylethoxy)carbonyl]-4-methylamino piperidine

Benzaldehyde (14.6 g, 0.138 mmol), 4-(aminomethyl)-piperdine (14.29 g, 0.125 mmol) and toluene (250 mL) were combined and heated at reflux for 4.5 h with removal of water. The reaction mixture was cooled to -25°C and di-*t*-butyl dicarbonate (28.7 g, 0.131) was added. The reaction mixture was allowed to warm to room temperature and stirred an additional 8 h. To this reaction mixture was added aqueous $KHSO_4$ (1.0 M, 120 mL) and stirred for 3 h. The organic layer was removed and the remaining aqueous layer was extracted with ether (3x75 mL). The aqueous layer was made basic through the addition of aqueous NaOH (1.0 M, 130 mL) and extracted with ether (3x75 mL). The combined organic extracts were dried over $NaSO_4$ filtered, and the solvent removed in vacuo to give title compound, 24.4 g (9 %), as a white solid.

B. N-[[(Phenylmethoxy)-carbonyl]-D-phenylalanyl]-L-proline methyl ester

To a solution of N-CBZ-D-phenylalanine (47.3 g, 0.158 mmol) in DMF (300 mL) at 0°C, was added L-proline-methylester.HCl (25.0 g, 0.151 mmol), 1-hydroxybenzotriazole hydrate (20.28 g, 0.166 mmol), ethyl-3-(3-dimethylamino)-propyl carbodiimide.HCl (31.8 g, 0.166 mmol) and 4-methylmorpholine (16.0 g, 0.159 mmol). The reaction mixture was stirred for 8 h while allowing the reaction to warm to room temperature. The reaction mixture was poured in water (1.2 L) and extracted with ethyl acetate (3x300 mL). The combined organic extracts were washed as follows: $KHSO_4$ (0.25 M, 2x200 mL), water (1x200 mL), saturated aqueous $NaHCO_3$ (2x200 mL) and saturated NaCl (1x200 mL). The organic layer was dried over $MgSO_4$, filtered and the solvent removed in vacuo to give title compound, 60.9 g (98%), as an oil.
MS $(M+H)^+ = 411^+$.

C. N-[[(Phenylmethoxy)-carbonyl]-D-phenylalanyl]-L-proline

To a solution of Part B compound (57.8 g, 0.141 mol) in MeOH (180 mL) and THF (60 mL) at 0°C was added aqueous NaOH (1.0 N, 183 mL). After 30 min the reaction mixture was warmed to room temperature and stirring continued for an additional 4 h. Aqueous HCl (1.0 N, 42 mL) was added and the organic solvents removed in vacuo. The resulting aqueous layer was acidified to pH of 2-3 and extracted with ethyl acetate (2x300 mL). The combined extracts were dried over $MgSO_4$, filtered and the solvent removed in vacuo to give title compound, 45.53 g (97 %), as a white solid. MS $(M+H)^+ = 397^+$.

D. N-[[[1-(1,1-Dimethylethoxy)carbonyl]-4-piperidinyl]methyl]-1-[N-[(phenylmethoxy)carbony]-D-phenylalanyl]-L-prolinamide

To a solution of Part C compound (19.39 g, 48.90 mmol) in DMF (120 mL) at 0 °C, was added Part A compound (9.98 g, 46.57 mmol), 1-hydroxybenzotriazole hydrate (6.26 g, 51.23 mmol) and ethyl-3-(3-dimethylamino)-propyl carbodiimide.HCl (9.82 g, 51.23 mmol). The reaction mixture was stirred for 12 h while allowing the reaction to warm to room temperature. The reaction mixture was poured in water (500 mL) and extracted with ethyl acetate (3x100 mL). The combined organic extracts were washed as follows: $KHSO_4$ (0.25 M, 2x50 mL), water (1x75 mL), saturated aqueous $NaHCo_3$ (2x75 mL) and saturated NaCl (1x50 mL). The organic layer was dried over $MgSO_4$, filtered and the solvent removed in vacuo to give title compound, 23.4 g (85 %), as an oil. MS $(M+H)^+ = 593^+$.

E. N-[[[[1-(1,1-Dimethylethoxy)carbonyl]-4-piperidinyl]methyl]-1-D-phenylalanyl]-L-prolinamide

To a stirred solution of Part D compound (15.00 g, 25.3 mmol) in MeOH (150 mL) was added Pd/C (1.50 g) and the reaction mixture was placed under 1 atmosphere of hydrogen. An additional Pd/C (2.5 g) was added after stirring for 2 h. The reaction was complete after stirring 8 more hours. The reaction mixture was filtered and the solvent removed in vacuo to give title compound, 11.41 g (91%), as a white solid. MS $(M+H)^+ = 459^+$.

F. N-[[[1-(1,1-Dimethylethoxy)carbonyl]-4-piperidinyl]methyl]-1-[N-[(2,2,2-trifluoroethyl)sulfonyl]-D-phenylalanyl]-L-prolinamide

To a solution of Part E compound (1.71 g, 3.5 mmol) and triethylamine (2.5 mL, 17.9 mmol) in chloroform (10 mL) at 0 °C under argon was added trifluoroethylsulfonyl chloride (0.5 mL, 4.5 mmol). The reaction mixture was stirred to room temperature overnight. After 24 hr, triethylamine (2.0 mL, 14.3 mmol) and trifluoroethylsulfonyl chloride (0.5 mL, 4.5 mmol) were added at 0 °C. The ice-water bath was removed, and the reaction was stirred for 6 h. The mixture was diluted with 0.25 M aqueous $KHSO_4$ and EtOAc. After separaton of the two layers, the organic layer was washed with 0.25 M aqueous $KHSO_4$, $H_2O$, saturated aqueous $NaHCO_3$, and $H_2O$, successively; dried over $NaSO_4$, filtered and concentrated in vacuo to give title compound, (1.60 g, 75 %):
MS $(M + H)^+ = 605^+$.

G. N-[[1-(Aminoiminomethyl)-4-piperidinyl]methyl]-1-[N-[(2,2,2-trifluoroethyl)sulfonyl]-D-phenylalanyl]-L-prolinamide, trifluoroacetate (1:1) salt

To a solution of Part F compound (1.60 g, 2.6 mmol) in dichloromethane (4.4 mL) at 0 °C under argon was added trifluoroacetic acid (3.0 mL, 38.9 mmol). After 5.5 h, the reaction mixture was concentrated in vacuo to give the corresponding TFA salt. To a solution of this salt (1.3 mmol) and 1H-pyrazole-1-carboxamidine hydrochloride (0.311 g, 2.1 mmol) in DMF (1.3 mL) under argon was added N,N-diisopropylethylamine (0.7 mL, 4.0 mmol). After 2 days, 1H-pyrazole-1-carboxamidine hydrochloride (0.150 g, 1.0 mmol) and N,N-diisopropylethylamine (0.5 mL, 2.9 mmol) were added. After 1 day, 1H-pyrazole-1-carboxamidine hydrochloride (0.150 g, 1.0 mmol) and N,N-diisopropylethylamine (0.5 mL, 2.9 mmol) were added. After 2 days, the reaction mixture was concentrated in vacuo and purified by preparative HPLC ($CH_3CN/H_2O$ with TFA buffer using a C-18 silica gel column). The appropriate fractions were combined, concentrated in vacuo, dissolved in $H_2O$ and lyophilized to yield title compound (0.256 g, 29 %) as a white solid:
$[a]_D$ = -56.0 (c 0.50, MeOH)
MS $(M + H)^+ = 547^+$

| Anal. Calc'd for $C_{23}H_{33}N_6O_4SF_3$ • 1.22 TFA • 1.14 $H_20$: | | | | | |
|---|---|---|---|---|---|
| | C, 43.26; | H, 5.21; | N, 11.90; | S, 4.54; | F, 17.92 |
| Found: | C, 42.82; | H, 4.87; | N, 11.48; | S, 4.98; | F, 17.52 |

Example 34

N-[[l-(Aminoiminomethyl)-4-piperidinyl]methyl]-l-[N-[(phenylmethyl)sulfonyl]-L-alanyl]-L-prolinamide, trifluoroacetate (l:l) salt

A. N-[[(t-Butoxy)-carbonyl]-L-alanyl]-L-proline methyl ester

To a solution of N-BOC-L-alanine (150 mmol) in DMF (250 mL) at 0°C, is added proline methyl ester.HCl (150 mmol), 1-hydroxybenzotriazole hydrate (22.2 g, 0.164 mmol), ethyl-3-(3-dimethylamino)-propyl carbodiimide.HCl (31.4 g, 0.164 mmol) and 4-methylmorpholine ( 22.6 g, 0.224 mmol). The reaction mixture is stirred for 12 h while allowing the reaction to warm to room temperature. The reaction mixture is poured in water (750 mL) and extracted with ethyl acetate (2x150 mL). The combined organic extracts are washed as follows: $KHSO_4$ (0.25 M, 2x50 mL), water (1x50 mL), saturated aqueous $NaHCO_3$ (2x50 mL) and saturated NaCl (1x50 mL). The organic layer is dried over $MgSO_4$, filtered and the solvent removed in vacuo to give title compound.

B. N-[[(Phenylmethyl)sulfonyl]carbonyl]-L-alanyl]-L-proline methyl ester

A solution of Part A compound (5.2 mmol) in trifluoroacetic acid (3 mL) is stirred at 0°C for 1.5 hr and and concentrated in vacuo to give the corresponding TFA salt. This salt is dissolved in chloroform (10 mL), and triethylamine (2.2 mL, 15.6 mmol) and benzylsulfonyl chloride (1.48, 7.8 mmol) at 0°C are added to it. The reaction mixture is stirred to room temperature overnight. After 25 h, the reaction mixture is poured into ETOAc (50 mL), washed with 0.25 M aqueous $KHSO_4$ (2x20 mL), $H_2O$ (1x20 mL) and saturated aqueous $NaHCO_3$ (2x20 mL), successively; dried over $MgSO_4$, filtered and concentrated in vacuo to give title compound.

C. N-[[(Phenylmethyl)sulfonyl]carbonyl]-L-alanyl]-L-proline

To a solution of Part B compound (3.75 mmole) in THF (10 mL) at 0°C is added aqueous NaOH (1.0 N, 9.5 mL). After 30 min the reaction mixture is warmed to room temperature and stirring continued for an additional 4 h. The organic solvent is removed in vacuo, the aqueous layer acidified to pH of 2-3 and extracted with ethyl acetate (3x10 mL). The combined extracts are dried over $MgSO_4$, filtered and the solvent removed in vacuo to give title compound.

D. N-[[[1-(1,1-Dimethylethoxy)carbonyl]-4-piperidinyl]methyl]-1-[N-[(phenylmethyl)sulfonyl]-L-alanyl]-L-prolinamide

To a solution of Part C compound (3.2 mmol) in DMF (15 mL) at 0°C is added N1-BOC-4-methylamino piperidine (3.2 mmol), 1-hydroxybenzotriazole hydrate (0.476 g, 3.5 mmol), ethyl-3-(3-dimethylamino)-propyl carbodiimide.HCl (0.675 g, 3.5 mmol) and 4-methylmorpholine until basic by pH paper. The reaction mixture is stirred for 12 h while allowing the reaction to warm to room temperature. The reaction mixture is poured into 0.25 M aqueous $KHSO_4$ (50 mL) and extracted with EtOAc (2x20 mL). The organic layers are combined and washed with 0.25 M aqueous $KHSO_4$ (1x30 mL), water (1x25 mL), saturated aqueous $NaHCO_3$ (2x25 mL) and water (1x20 mL), the organic layer dried over $MgSO_4$, filtered and the solvent removed in vacuo to give title compound.

E. N-[[1-(Aminoiminomethyl)-4-piperidinyl]methyl]-1-[N-[(phenylmethyl)sulfonyl]-L-alanyl]-L-prolinamide, trifluoroacetate (1:1) salt

A solution of Part D compound (2. 2 mmol) in trifluoroacetic acid (10.0 mL) is stirred at 0°C for 5 h. The reaction mixture is concentrated in vacuo to give the corresponding TFA salt. To a solution of this salt (2.2 mmol), 1H-pyrazole-1-carboxamidine hydrochloride (0.474 g, 3.3 mmol) and N,N-diisopropylethylamine (1.1 mL, 6.5 mmol) in DMF (3.0 mL) are added and stirred for 3 days. The reaction mixture is concentrated in vacuo and purified by preparative HPLC ($CH_3CN/H_2O$ with TFA buffer using a C-18 silica gel column). The appropriate fractions are combined, concentrated in vacuo, dissolved in $H_2O$ and lyophilized to yield title compound.

Example 35

N-[[l-(Aminoiminomethyl)-4-piperidinyl]methyl]-l-[N-[(phenylmethyl)sulfonyl]-glycyl]-L-prolinamide, trifluoroacetate (l:l) salt

A. N-[[(t-Butoxy)-carbonyl]-glycyl]-L-proline methyl ester

To a solution of N-BOC-glycine (150 mmol) in DMF (250 mL) at 0°C, is added proline methyl ester.HCl (150 mmol), 1-hydroxybenzotriazole hydrate (22.2 g, 0.164 mmol), ethyl-3-(3-dimethylamino)propyl carbodiimide.HCl (31.4 g, 0.164 mmol) and 4-methylmorpholine ( 22.6 g, 0.224 mmol). The reaction mixture is stirred for 12 h while allowing the reaction to warm to room temperature. The reaction mixture is poured in water (750 mL) and extracted with ethyl acetate (2x150 mL). The combined organic extracts are washed as follows: $KHSO_4$ (0.25 M, 2x50 mL), water (1x50 mL), saturated aqueous $NaHCO_3$ (2x50 mL) and saturated NaCl (1x50 mL). The organic layer is dried over $MgSO_4$, filtered and the solvent removed in vacuo to give title compound.

B. N-[[[(Phenylmethyl)sulfonyl]carbonyl]glycyl]-L-proline methyl ester

A solution of Part A compound (5.2 mmol) in trifluoroacetic acid (3 mL) is stirred at 0°C for 1.5 hr and and concentrated in vacuo to give the corresponding TFA salt. This salt is dissolved in chloroform (10 mL), and triethylamine (2.2 mL, 15.6 mmol) and benzylsulfonyl chloride (1.48, 7.8 mmol) at 0°C are added to it. The reaction mixture is stirred to room temperature overnight. After 25 h, the reaction mixture is poured into EtOAc (50 mL), washed with 0.25 M aqueous $KHSO_4$ (2x20 mL), $H_2O$ (1x20 mL) and saturated aqueous $NaHCO_3$ (2x20 mL), successively; dried over $MgSO_4$, filtered and concentrated in vacuo to give title compound.

C. N-[[[(Phenylmethyl)sulfonyl]carbonyl]glycyl]-L-proline

To a solution of Part B compound (3.75 mmole) in THF (10 mL) at 0°C is added aqueous NaOH (1.0 N, 9.5 mL). After 30 min the reaction mixture is warmed to room temperature and stirring continued for an additional 4 h. The organic solvent is removed in vacuo, the aqueous layer acidified to pH of 2-3 and extracted with ethyl acetate (3x10 mL). The combined extracts are dried over $MgSO_4$, filtered and the solvent removed in vacuo to give title compound.

D. N-[[[1-(1,1-Dimethylethoxy)carbonyl]-4-piperidinyl]methyl]-1-[N-[(phenylmethyl)sulfonyl]-glycyl]-L-prolinamide

To a solution of Part C compound (3.2 mmol) in DMF (15 mL) at 0°C is added N1-BOC-4-methylamino piperidine (3.2 mmol), 1-hydroxybenzotriazole hydrate (0.476 g, 3.5 mmol), ethyl-3-(3-dimethylamino)-propyl carbodiimide.HCl (0.675 g, 3.5 mmol) and 4-methylmorpholine until basic by pH paper. The reaction mixture is stirred for 12 h while allowing the reaction to warm to room temperature. The reaction mixture is poured into 0.25 M aqueous $KHSO_4$ (50 mL) and extracted with EtOAc (2x20 mL). The organic layers are combined and washed with 0.25 M aqueous $KHSO_4$ (1x30 mL), water (1x25 mL), saturated aqueous $NaHCO_3$ (2x25 mL) and water (1x20 mL), the organic layer dried over $MgSO_4$, filtered and the solvent removed in vacuo to give title compound.

E. N-[[1-(Aminoiminomethyl]-4-piperidinyl]methyl]-1-[N-[(phenylmethyl)sulfonyl]-glycyl]-L-prolinamide, trifluoroacetate (1:1) salt

A solution of Part D compound (2. 2 mmol) in trifluoroacetic acid (10.0 mL) is stirred at 0°C for 5 h. The reaction mixture is concentrated in vacuo to give the corresponding TFA salt. To a solution of this salt (2.2 mmol), 1H-pyrazole-1-carboxamidine hydrochloride (0.474 g, 3.3 mmol) and N,N-diisopropylethylamine (1.1 mL, 6.5 mmol) in DMF (3.0 mL) are added and stirred for 3 days. The reaction mixture is concentrated in vacuo and purified by preparative HPLC ($CH_3CN/H_2O$ with TFA buffer using a C-18 silica gel column). The appropriate fractions are combined, concentrated in vacuo, dissolved in $H_2O$ and lyophilized to yield title compound.

Following the procedures of Example 30 to 33, the following examples of compounds of the invention may be prepared.

TABLE

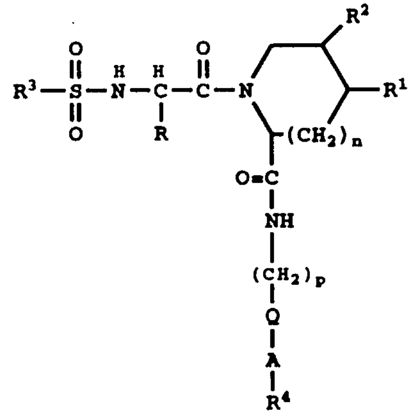

| Example No. | $R^3$ | R | $R^2$ | $R^1$ | n | p | Q | A | $R^4$ |
|---|---|---|---|---|---|---|---|---|---|
| 36 | $CH_3$ | $CH_2OH$(S) | H | $CH_3$ | 1 | 0 | - | 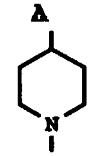 | 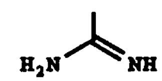 |
| 37 | $C_6H_5$ | H | OH | H | 0 | 1 | CO | 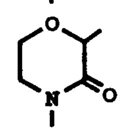 | 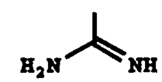 |
| 38 | 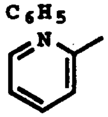 | $-CH_2C_6H_5$(R) | $OCH_3$ | $CH_3$ | 0 | 2 | - | 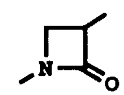 | 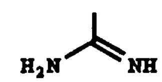 |

| Example No. | $R^3$ | R | $R^2$ | $R^1$ | n | p | Q | A | $R^4$ |
|---|---|---|---|---|---|---|---|---|---|
| 39 | 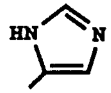 | $-CH_2C_6H_5(S)$ | | | | | | | |
| 40 | 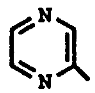 | $-CH_2CH_2CONH_2(S)$ | H | H | 1 | 0 | - | 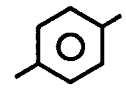 | $-CH_2NH_2$ |
| 41 | $C_6H_5$ | $-CH_2CH_2CONH_2(R)$ | H | $CH_3$ | 1 | 1 | CO | 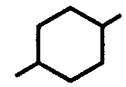 | $-CH_2NH_2$ |
| 42 | 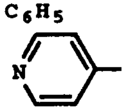 | $-CH(OH)CH_3(S\text{-}Thr)$ | $-CHCH_2CH_2CH-$ | | 0 | 2 | - | 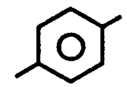 | 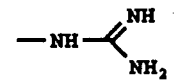 |
| 43 | 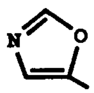 | $CH(OH)CH_3(S\text{-}alloThr)$ | $CH_3$ | H | 1 | 2 | CO | 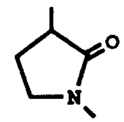 | 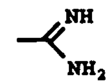 |
| 44 | 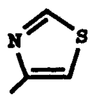 | 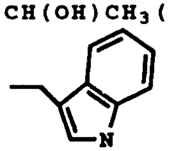 (R) | $SCH_3$ | $CH_3$ | 1 | 1 | - | 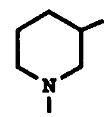 | 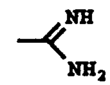 |
| 45 | 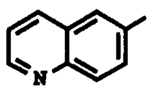 | $-CH_2CH_2CO_2H(R)$ | H | $CH_3$ | 0 | 0 | CO | 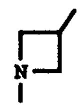 | 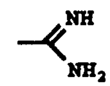 |

| Example No. | $R^3$ | R | $R^2$ | $R^1$ | n | p | Q | A | $R^4$ |
|---|---|---|---|---|---|---|---|---|---|
| 46 | $C_2H_5$ | $CH_2OCH_2Ph(R)$ | H | H | 1 | 0 | - | 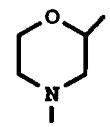 | 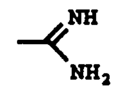 |
| 47 | $C_6H_5CH_2$ | $CH_2CH_2Ph(S)$ | H | H | 1 | 1 | - | 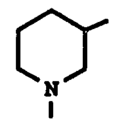 | 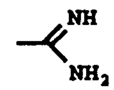 |
| 48 | 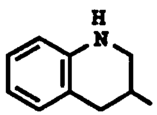 | 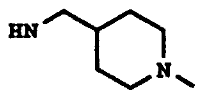 | H | H | 1 | 2 | - | 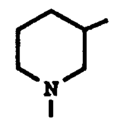 | 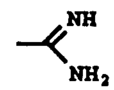 |

Example 49

N-[[l-(Aminoiminomethyl)-4-piperidinyl]methyl]-l-[N-(ethylsulfonyl)-D-phenylalanyl]-L-prolinamide, trifluoroacetate (l:l) salt

$[\alpha]_D$ = -49.2 (c l.08, MeOH)
MS $(M + H)^+ = 493^+$

| Anal. Calc'd for $C_{23}H_{36}N_6O_4S$ • l.4 TFA • l.0 $H_2O$: | | | | | |
|---|---|---|---|---|---|
| | C, 46.23; | H, 5.92; | N, l2.54; | S, 4.78; | F, ll.90 |
| Foudn: | C, 46.23; | H, 6.08; | N, l2.39; | S, 4.7l; | F, ll.98 |

Example 50

N-[[l-(Aminoiminomethyl)-4-piperidinyl]methyl]-l-[N-(propylsulfonyl)-D-phenylalanyl]-L-prolinamide, trifluoroacetate (l:l) salt

$[\alpha]_D$ = -47.0 (c l.02, MeOH)
MS $(M + H)^+ = 507^+$

| Anal. Calc'd for $C_{24}H_{38}N_6O_4S$ • l.l5 TFA • l.2 $H_2O$: | | | | | |
|---|---|---|---|---|---|
| | C, 47.9l; | H, 6.35; | N, l2.74; | S, 4.86; | F, 9.94 |
| Found: | C, 47.90; | H, 6.2l; | N, l2.50; | S, 4.82; | F, l0.06 |

Example 5l

N-[[l-(Aminoiminomethyl)-4-piperidinyl]methyl]-l-[N-[(phenylmethyl)sulfonyl]-D-phenylalanyl]-L-prolinamide, trifluoroacetate (l:l) salt

$[\alpha]_D$ = -44.6 (c 0.8, MeOH)
MS $(M + H)^+ = 555^+$

| Anal. Calc'd for $C_{28}H_{38}N_6O_4S$ • l.55 TFA • 0.67 $H_2O$: | | | | | |
|---|---|---|---|---|---|
| | C, 50.3l; | H, 5.42; | N, ll.32; | S, 4.32; | F, ll.90 |
| Found: | C, 50.3l; | H, 5.69; | N, ll.24; | S, 4.26; | F, ll.97 |

Example 52

N-[[l-(Aminoiminomethyl)-4-piperidinyl]methyl]-l-[N-(phenylsulfonyl)-D-phenylalanyl]-L-prolinamide, trifluoroacetate (l:l) salt

$[\alpha]_D$ = +l0.l (c l.0, MeOH)
MS $(M + H)^+ = 54l^+$

| Anal. Calc'd for $C_{27}H_{36}N_6O_4S$ • l.3 TFA • 0.83 $H_2O$: | | | | | |
|---|---|---|---|---|---|
| | C, 50,5l; | H, 5.58; | N, ll.94; | S, 4.55; | F, l0.53 |
| Found: | C, 50.5l; | H, 5.47; | N, ll.83; | S, 4.7l; | F, l0.52 |

Example 53

N-[[l-(Aminoiminomethyl)-4-piperidinyl]methyl]-l-[N-[(l-methylethyl)sulfonyl]-D-phenylalanyl]-L-prolinamide, trifluoroacetate (2:3) salt

$[\alpha]_D$ = -43.l (c l.l0, MeOH)
MS $(M + H)^+ = 507^+$

| Anal. Calc'd for $C_{24}H_{38}N_6O_4S$ • l.5 TFA • l.35 $H_2O$: | | | | | |
|---|---|---|---|---|---|
| | C, 46.20; | H, 6.06; | N, ll.97; | S, 4.57; | F, l2.l8 |
| Found: | C, 46.6l; | H, 5.86; | N, ll.95; | S, 4.44; | F, l2.02 |

## Claims

**1.** A compound having the structure

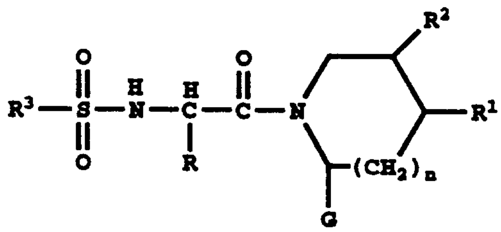

including all stereoisomers, wherein n is 0, l or 2;

G is an amido moiety which includes a cyclic member;

R is hydrogen, hydroxyalkyl, aminoalkyl, alkyl, cycloalkyl, arylalkyl, alkenyl, alkynyl, amidoalkyl, arylalkoxyalkyl or an amino acid side chain, either protected or unprotected;

$R^1$ and $R^2$ are independently hydrogen, lower alkyl, cycloalkyl, aryl, hydroxy, alkoxy, keto, thioketal, thioalkyl, thioaryl, amino or alkylamino; or $R^1$ and $R^2$ together with the carbons to which they are attached form a cycloalkyl, aryl or heteroaryl ring;

$R^3$ is alkyl, arylalkyl, aryl, heteroaryl, quinolinyl or tetrahydroquinolinyl;

including pharmaceutically acceptable salts thereof.

**2.** The compound as defined in Claim l wherein G is

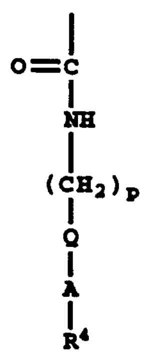

wherein p is 0, l or 2;

Q is a single bond or

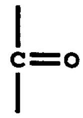 ;

A is aryl or cycloalkyl or an azacycloalkyl ring of 4 to 8 carbons in the ring or an azaheteroalkyl ring of 4 to 8 carbons in the ring of the structure

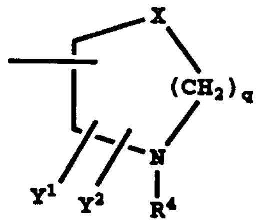

where X is $CH_2$, O, S or NH;

q is 0, l, 2, 3 or 4, provided that

q is 0, l, 2, 3 or 4 if X is $CH_2$;

q is 2, 3 or 4 if X is O, S or NH;

$Y^1$ and $Y^2$ are independently H, lower alkyl, halo or keto;

$R^4$ is guanidino, amidino or aminomethyl; with the provisos that where A is aryl or cycloalkyl, $R^4$ is guanidino, amidino or aminomethyl,

where A is azacycloalkyl or azaheteroalkyl, $R^4$ is amidino;

where A is azaheteroalkyl, then there must be at least a 2-carbon chain between X and any N atom in the ring or outside the ring.

**3.** The compound as defined in Claim l wherein A is aryl or cycloalkyl, Q is a single bond and q is 0 or l.

**4.** The compound as defined in Claim l wherein A is

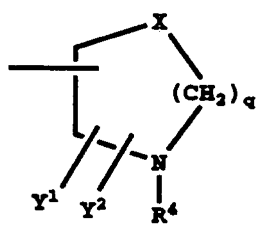 .

**5.** The compound as defined in Claim 4 wherein X is $CH_2$ or NH and $Y^1$ and $Y^2$ are each H.

**6.** The compound as defined in Claim 4 wherein G is

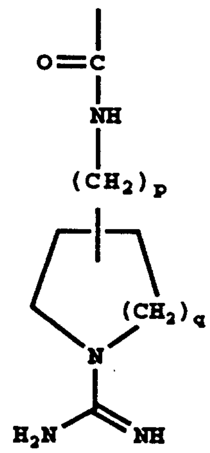

X is $CH_2$, q is 0 or l,
$R^1$ and $R^2$ are each H, $R^3$ is alkyl, arylalkyl or aryl, n is 0 or l and R is aralkyl or hydroxyalkyl.

**7.** The compund as defined in Claim I which is N-[[l-(aminoiminomethyl)-4-piperidinyl]methyl]-l-[N-(methylsulfonyl)-D-phenylalanyl]-L-prolinamide or a salt thereof;
N-[[l-(aminoiminomethyl)-3-piperidinyl]methyl]-l-[N-(methylsulfonyl)-D-phenylalanyl]-L-prolinamide or a salt thereof;
N-[[l-(aminoiminomethyl)-4-piperidinyl]methyl]-l-[N-[(phenylmethyl)sulfonyl]-D-alanyl]-L-prolinamide, or salt thereof or its trifluoroacetate (l:l) salt;
N-[[l-(aminoiminomethyl)-4-piperidinyl]methyl]-l-[N-[(2,2,2-trifluoroethyl)sulfonyl]-D-phenylalanyl]-L-prolinamide, or salt thereof or its trifluoroacetate (l:l) salt;
N-[[l-(aminoiminomethyl)-4-piperidinyl]methyl]-l-[N-[(phenylmethyl)sulfonyl]-L-alanyl]-L-prolinamide, or salt thereof or its trifluoroacetate (l:l) salt;
N-[[l-(aminoiminomethyl)-4-piperidinyl]methyl]-l-[N-[(phenylmethyl)sulfonyl]glycyl]-L-prolinamide, or salt thereof or its trifluoroacetate (l:l) salt;
N-[[l-(aminoiminomethyl)-4-piperidinyl]methyl]-l-[N-(ethylsulfonyl)-D-phenylalanyl]-L-prolinamide, or salt thereof or its trifluoroacetate (l:l) salt;
N-[[l-(aminoiminomethyl)-4-piperidinyl]methyl]-l-[N-(propylsulfonyl)-D-phenylalanyl]-L-prolinamide, or salt thereof or its trifluoroacetate (l:l) salt;
N-[[l-(aminoiminomethyl)-4-piperidinyl]methyl]-l-[N-[(phenylmethyl)sulfonyl]-D-phenyl alanyl]-L-prolinamide, or salt thereof or its trifluoroacetate (l:l) salt;
N-[[l-(aminoiminomethyl)-4-piperidinyl]methyl]-l-[N-(phenylsulfonyl)-D-phenylalanyl]-L-prolinamide, or salt thereof or its trifluoroacetate (l:l) salt;
N-[[l-(aminoiminomethyl)-4-piperidinyl]methyl]-l-[N-[(l-methylethyl)sulfonyl]-D-phenylalanyl]-L-prolinamide, or salt thereof or its trifluoroacetate (2:3) salt.

**8.** A compound having the structure

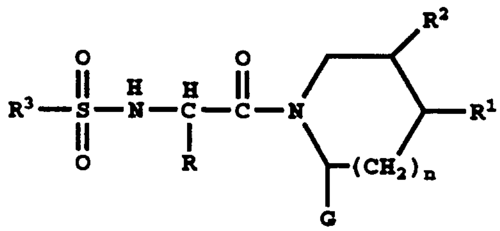

including all stereoisomers, wherein n is 0, l or 2;
G is an amido moiety of the structure

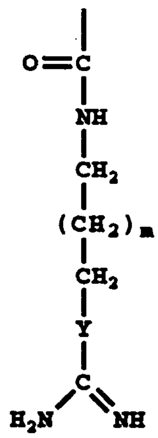

wherein m is 0, l, 2 or 3; and

Y is NH or S;

R is hydrogen, hydroxyalkyl, aminoalkyl, alkyl, cycloalkyl, arylalkyl, alkenyl, alkynyl, amidoalkyl, arylalkoxyalkyl or an amino acid side chain, either protected or unprotected;

$R^1$ and $R^2$ are independently hydrogen, lower alkyl, cycloalkyl, aryl, hydroxy, alkoxy, keto, thioketal, thioalkyl, thioaryl, amino or alkylamino; or $R^1$ and $R^2$ together with the carbons to which they are attached form a cycloalkyl, aryl or heteroaryl ring;

$R^3$ is alkyl, arylalkyl, aryl, heteroaryl, quinolinyl or tetrahydroquinolinyl;

provided that where $R^3$ is alkyl, the alkyl has at least 3 carbons;

including pharmaceutically acceptable salts thereof.

**9.** The compound as defined in Claim 8 having the structure

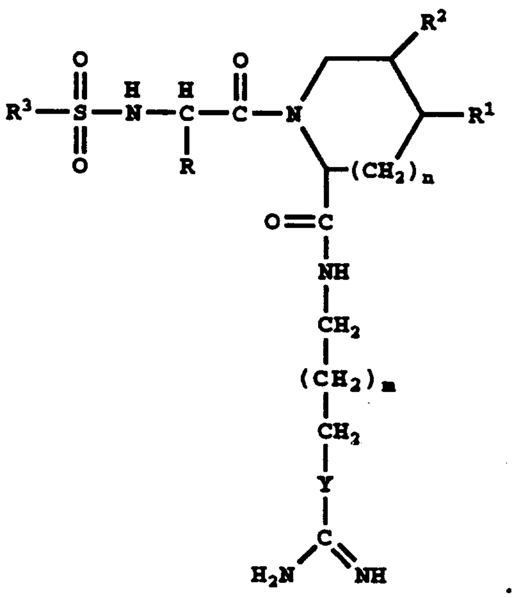

**10.** The compound as defined in Claim 9 wherein n is l and m is 2, $R^3$ is 2-naphthyl, lower alkyl having at least 3 carbons, or benzyl, R is hydroxyalkyl or aralkyl, $R^1$ is H or alkyl, $R^2$ is H and Y is -NH-.

**11.** The compound as defined in Claim 9 having the structure

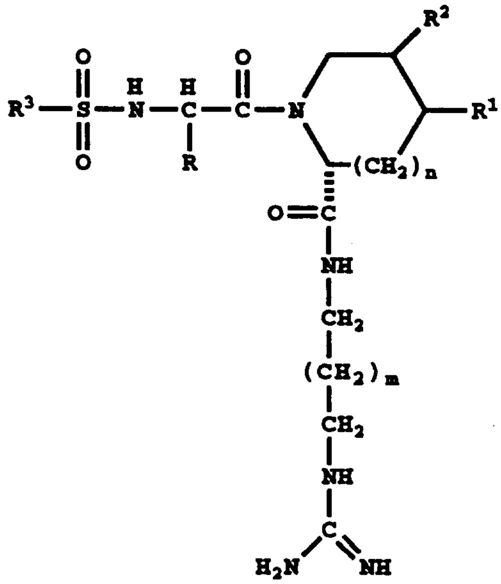

**12.** The compound as defined in Claim 9 which is [l(S),2α,4β]-N-[4-[(aminoiminomethyl)amino]butyl]-l-[3-hydroxy-2-[(2-naphthalenylsulfonyl)amino]-l-oxopropyl]-4-methyl-2-piperidinecarboxamide, or a salt thereof or its trifluoroacetate (l:l) salt;

[l(S),2α,4β]-N-[4-[(aminoiminomethyl)amino]butyl]-4-methyl-l-[2[(2-naphthalenylsulfonyl)amino]-l-oxo-3-(phenylmethoxy)propyl]-2-piperidinecarboxamide, or a salt thereof or its trifluoroacetate (l:l) salt;

[2R-[l(S*),2α,4β]-N-[4-[(aminoiminomethyl)amino]butyl]-l-[3-hydroxy-2-[(2-naphthalenylsulfonyl)-amino]-l-oxopropyl]-4-methyl-2-piperidinecarboxamide, or a salt thereof or its trifluoroacetate (l:l) salt;

[2S-[l(R*),2α,4β]-N-[4-[(aminoiminomethyl)amino]butyl]-l-[3-hydroxy-2-[(2-naphthalenylsulfonyl)-amino]-l-oxopropyl]-4-methyl-2-piperidinecarboxamide, or a salt thereof or its trifluoroacetate (l:l) salt;

(2S-trans)-N-[4-[(aminoiminomethyl)amino]butyl[-4-methyl-l-[[[(l,2,3,4-tetrahydro-3-methyl-8-quinolinyl)sulfonyl]amino]acetyl]-2-piperidinecarboxamide, or a salt thereof or its trifluoroacetate salt;

trans-N-[4-[(aminoiminomethyl)amino]butyl]-4-(methylthio)-l-[[(2-naphthalenylsulfonyl)amino]acetyl]-L-prolinamide, or a salt thereof or its trifluoroacetate salt;

N-[4-[(aminoiminomethyl)amino]butyl]-l-[N-(2-naphthalenylsulfonyl)-D-phenylalanyl]-L-prolinamide, or a salt thereof or its trifluoroacetate salt, hemihydrate;

N-[4-[(aminoiminomethyl)amino]butyl]-l-[N-(2-naphthalenylsulfonyl)-D-glutaminyl]-L-prolinamide or a salt thereof or its trifluoroacetate salt;

[2R-[l(S*),2α,4β)]-N-[3-[(aminoiminomethyl)amino]-propyl]-l-[4-hydroxy-3-[(2-naphthalenylsulfonyl)-amino)-l,2-dioxobutyl]-2-piperidinecarboxamide, or a salt thereof, or its trifluoroacetate (l:l) salt;

N-[4-[(aminoiminomethyl)amino]butyl]-l-[N-(2-naphthalenylsulfonyl)-L-seryl]-L-prolinamide or a salt thereof, or its trifluoroacetate (l:l) salt;

N-[4-[(aminoiminomethyl)amino]butyl]-l-[N-(2-naphthalenylsulfonyl)-L-seryl]-D-prolinamide, or a salt thereof, or its trifluoroacetate (l:l) salt;

(2R-trans)-N-[4-[(aminoiminomethyl)amino]butyl]-4-methyl-l-[[(2-naphthalenylsulfonyl)amino]acetyl]-2-piperidinecarboxamide, or a salt thereof, or its trifluoroacetate salt, hydrate;

[2R-[l(S*)2α,4β]]-N-[4-[(aminoiminomethyl)amino]butyl]-l-[3-hydroxy-2-[(2-naphthalenylsulfonyl)-amino]-l-oxopropyl]-4-methyl-2-piperidinecarboxamide, or a salt thereof, or its trifluoroacetate salt, hydrate;

[l(S)]-N-[4-[(aminoiminomethyl)amino]butyl]-l,2,3,4-tetrahydro-2-[3-hydroxy-2-[(2-naphthalenylsulfonyl)amino]-l-oxopropyl]-3-isoquinolinecarboxamide, isomer A, or a salt thereof, or its trifluoroacetate salt, hydrate;

[l(S)]-N-[4-[(aminoiminomethyl)amino]butyl]-1,2,3,4-tetrahydro-2-[3-hydroxy-2-[(2-naphthalenylsulfonyl)amino]-l-oxopropyl]-3-isoquinolinecarboxamide, isomer B, or a salt thereof, or its trifluoroacetate salt, hydrate;

(4S*)-N-[4-[(aminoiminomethyl)amino]butyl]-4-hydroxy-l-[N-(2-naphthalenylsulfonyl)-L-seryl]-L-prolinamide, or a salt thereof, or its trifluoroacetate (l:l) salt;

(4S*)-N-[4-[(aminoiminomethyl)amino]butyl]-4-methoxy-l-[N-(2-naphthalenylsulfonyl)-L-seryl]-L-prolinamide, or a salt thereof, or its trifluoroacetate (l:l) salt;

N-[4-[(aminoiminomethyl)amino]butyl]-l-[N-(2-naphthalenylsulfonyl)-L-phenylalanyl]-L-prolinamide, or a salt thereof, or its trifluoroacetate (l:l) salt;

(2S-trans)-N-[4-[(aminoiminomethyl)amino]butyl]-4-methyl-l-[[(2-naphthalenylsulfonyl)amino] acetyl]-2-piperidinecarboxamide or a salt thereof, or its trifluoroacetate (l:l)salt;

N-[4-[(aminoiminomethyl)amino]butyl]-l-[N-(2-naphthalenylsulfonyl)-L-glutaminyl]-L-prolinamide, or a salt thereof, or its trifluoroacetate (l:l) salt;

N-[4-[(aminoiminomethyl)amino]butyl]-l-[N-(2-naphthalenylsulfonyl)-L-threonyl]-L-prolinamide, or a salt thereof, or its trifluoroacetate (l:l) salt;

N-[4-[(aminoiminomethyl)amino]butyl]-l-[N-(2-naphthalenylsulfonyl)-L-allothreonyl]-L-prolinamide, or a salt thereof, or its trifluoroacetate (l:l) salt;

(S)-N-[4-[(aminoiminomethyl)amino]butyl]-4-(methylthio)-l-[N-(2-naphthalenylsulfonyl)glycyl]-L-prolinamide, or a salt thereof, or its trifluoroacetate (l:l) salt;

(R)-N-[4-[(aminoiminomethyl)amino]butyl]-4-(methylthio)-l-[N-(2-naphthalenylsulfonyl)glycyl]-L-prolinamide, or a salt thereof, or its trifluoroacetate (l:l) salt;

N-[4-[(aminoiminomethyl)amino]butyl]-l-[[(2-naphthalenylsulfonyl)amino]acetyl]-2-piperidinecarboxamide, or a salt thereof, or its trifluoroacetate (l:l) salt;

N-[4-[(aminoiminomethyl)amino]butyl]-l-[N-(2-naphthalenylsulfonyl)-D-tryptophyl]-L-prolinamide, or a salt thereof, or its trifluoroacetate (l:l) salt;

(2S-trans)-N-[4-[(aminoiminomethyl)amino]butyl]-4-methyl-l-[[[(3-methyl-8-quinolinyl)sulfonyl]amino]-acetyl]-2-piperidinecarboxamide, or a salt thereof, or its trifluoroacetate salt;

N-[4-[(aminoiminomethyl)amino]butyl]-l-[[(8-quinolinylsulfonyl)amino]acetyl]-2-piperidinecarbox amide, or a salt thereof, or its trifluoroacetate salt;

N-[4-[(aminoiminomethyl)amino]butyl]-l-[N-(2-naphthalenylsulfonyl)-D-α-glutamyl]-L-prolinamide, or a salt thereof, or its trifluoroacetate (l:l) salt; or

N-[4-[(aminoiminomethyl)amino]butyl]-l-[N-(methyl-sulfonyl)-D-phenylalanyl]-L-prolinamide or a salt thereof or its trifluoroacetate (l:l) salt.

**13.** A method of inhibiting or preventing formation of blood clots, which comprises administering to a patient in need of treatment a therapeutically effective amount of a compound as defined in Claim l.

**14.** The use of a sulfonamido heterocyclic compound or a pharmaceutical composition containing a sulfonamido heterocyclic compound for inhibiting or preventing blood clots, which comprises employing a compound as defined in Claim 1 or Claim 8.